# EUROPEAN PATENT SPECIFICATION

(11) **EP 0 771 352 B1**
(45) Date of publication and mention of the grant of the patent: **11.01.2006**
(21) Application number: 95926676.8
(22) Date of filing: 13.07.1995
(51) Int. Cl.: C12N 15/31, C07K 14/285, A61K 39/102, C12N 1/21, C12N 5/10, C12Q 1/68

(54) **NONTYPABLE HAEMOPHILUS INFLUENZAE LKP TIP ADHESIN PROTEIN AND NUCLEIC ACID**
HAEMOPHILUS INFLUENZAE (NT HI) LKP TIP ADHESIN PROTEIN UND NUKLEINSÄURE
LKP TIP ADHESIN PROTEINE ET ACIDE NUCLEIQUE DE HAEMOPHILUS INFLUENZAE INCLASSABLE

(30) Priority: 19.07.1994 US 277231; 07.06.1995 US 473750; 07.06.1995 US 477326
(43) Date of publication of application: 07.05.1997
(73) Proprietor: Wyeth Holdings Corporation, Madison, NJ 07940 (US); BACTEX, INC., Pittsburgh, PA 15213 (US)
(72) Inventor: GREEN, Bruce, A., Pittsford, NY 14534 (US); BRINTON, Charles, C., Jr., Export, PA 15632 (US)
(74) Representative: Hally, Anna-Louise
(86) International application number: PCT/US1995/008789
(87) International publication number: WO 1996/002648

(56) References cited:
- WO-A-93/19090
- US-A- 5 336 490
- Database EMBL/GenBank/DDBJ on STRAND,ID=HIIFC, AC=U02932, Watson et al: "Identification of a Gene essential for Piliation in Haemophilus influenzae type b with Homology to the Pilus Assembly Platform Genes of Gram-Negative Bacteria", 1994.
- Database EMBL/GenBank/DDBJ on STRAND,ID=HIHIFB, AC=X66606, Smith AL et al: "hif B of H. influenzae is a member of the chaperone family", 1992.
- Database EMBL/GenBank/DDBJ on STRAND,ID=HIHIIFA, AC=X16991, van Ham SM et al: "Cloning and expressionin Escherichia coli of Haemophilus influenzae fimbral genes establisches adherence to oropharyn- geal epithelial cells", EMBO J., vol 8,p3535, 1989.
- Database EMBL/GenBank/DDBJ on STRAND, ID=HII9795, AC=U19795, Green BA et al: "Sequence of LKP serotype 5 hifA gene", 1995.

### SEQUENCE LISTING

(1) GENERAL INFORMATION:
   (i) APPLICANT: Wyeth Holdings Coporation
      (A) NAME: Wyeth Holdings Coporation
      (B) STREET: Five Giralda Farms
      (C) CITY: Madison
      (D) STATE/PROVINCE: New Jersey
      (E) COUNTRY: USA
      (F) POSTAL CODE/ZIP: 07940
   (i) APPLICANT:
      (A) NAME: Bactex Inc.
      (B) STREET: 4516 Henry Street
      (C) CITY: Pittsburgh
      (D) STATE/PROVINCE: Pennsylvania
      (E) COUNTRY: USA
      (F) POSTAL COPE/ZIP: 15213
   (i) INVENTOR:
      (A) NAME: Bruce A. Green
      (B) STREET: 40 Northfield Gate
      (C) CITY: Pittsford
      (D) STATE/PROVINCE: New York
      (E) COUNTRY: USA
      (P) POSTAL CODE/ZIP: 14534
   (i) INVENTOR:
      (A) NAME: Charles C. Brinton, Jr.
      (B) STREET: 4913 Simmons Drive
      (C) CITY: Export
      (D) STATE/PROVINCE: Pennsylvania
      (E) COUNTRY: USA
      (F) POSTAL CODE/ZIP: 15632
   (ii) TITLE OF INVENTION:
      Nontypable Haemophilus Influenzae LKP Tip Adhesin Protein and Nucleic Acid
   (iii) NUMBER OF SEQUENCES: 21
   (iv) CORRESPONDENCE ADDRESS:
      (A) ADDRESSEE: Hamilton, Brook, Smith & Reynolds, P.C.
      (B) STREET: 530 Virginia Road, P.O. Box 9133
      (C) CITY: Concord
      (D) STATE: Massachusetts
      (E) COUNTRY: US
      (F) ZIP: 01742-9133
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30
   (vi) CURRENT APPLICATION DATA:
      (A) APPLICATION NUMBER: PCT/US95/08789
      (B) FILING DATE: 13 July 1995
      (C) CLASSIFICATION:
   (vii) PRIOR APPLICATION DATA:
      (A) APPLICATION NUMBER: US 08/277,321
      (B) FILING DATE: 19-JUL-1994
   (vii) PRIOR APPLICATION DATA:
      (A) APPLICATION NUMBER: US 08/473,750
      (B) FILING DATE: 07-JUN-1995
   (viii) ATTORNEY/AGENT INFORMATION:
      (A) NAME: Carroll, Alice O.
      (B) REGISTRATION NUMBER: 33,542
      (C) REFERENCE/DOCKET NUMBER: ACC94-02BA PCT
   (ix) TELECOMMUNICATION INFORMATION:
      (A) TELEPHONE: 617-861-6240
      (B) TELEFAX: 617-861-9540
(2) INFORMATION FOR SEQ ID NO:1:
   (i) SEQUENCE CHARACTERISTICS:
      (A) LENGTH: 217 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:1:
   Met Glu Gln Phe Ile Met Lys Lys Thr Thr Thr Gly Ser Leu Ile Leu
   1 5 10 15
   Leu Ala Phe Ala Thr Asn Ala Ala Asp Pro Gln Val Ser Thr Glu Thr
   20 25 30
   Ser Gly Lys Val Thr Phe Phe Gly Lys Val Val Glu Asn Thr Cys Lys
   35 40 45
   Val Lys Thr Asp Ser Lys Asn Met Ser Val Val Leu Asn Asp Val Gly
   50 55 60
   Lys Asn His Leu Lys Thr Lys Lys Asp Thr Ala Met Pro Thr Pro Phe
   65 70 75 80
   Thr Ile Asn Leu Glu Asn Cys Ser Thr Thr Thr Thr Thr Asn Asn Lys
   85 90 95
   Pro Val Ala Thr Lys Val Gly Ala Tyr Phe Tyr Ser Trp Lys Asn Ala
   100 105 110
   Asp Glu Asn Asn Glu Tyr Thr Leu Lys Asn Thr Lys Ser Gly Asn Asp
   115 120 125
   Ala Ala Gln Asn Val Asn Ile Gln Thr Phe Asp Ala Asn Gly Thr Asp
   130 135 140
   Ala Ile Glu Val Val Gly Asn Gly Thr Thr Asp Phe Thr His Ser Asn
   145 150 155 160
   Thr Asn Asp Val Ala Thr Gln Gln Thr Val Asn Lys Asn His Ile Ser
   165 170 175
   Gly Lys Ala Thr Ile Asn Gly Glu Asn Asn Val Lys Leu His Tyr Ile
   180 185 190
   Ala Arg Tyr Tyr Ala Thr Ala Gln Ala Glu Ala Gly Lys Val Glu Ser
   195 200 205
   Ser Val Asp Phe Gln Ile Ala Tyr Glu
   210 215
(2) INFORMATION FOR SEQ ID NO:2:
   (i) SEQUENCE CHARACTERISTICS:
      (A) LENGTH: 216 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:2:
   Met Glu Gln Phe Ile Met Lys Lys Thr Leu Leu Gly Ser Leu Ile Leu
   1               5                   10                  15
   Leu Ala Phe Ala Gly Asn Val Gln Ala Asp Ile Asn Thr Glu Thr Ser
               20                  25                  30
   Gly Lys Val Thr Phe Phe Gly Lys Val Val Glu Asn Thr Cys Lys Val
           35                  40                  45
   Lys Thr Glu His Lys Asn Leu Ser Val Val Leu Asn Asp Val Gly Lys
       50                  55                  60
   Asn Ser Leu Ser Thr Lys Val Asn Thr Ala Met Pro Thr Pro Phe Thr
   65                  70                  75                  80
   Ile Thr Leu Gln Asn Cys Asp Pro Thr Thr Ala Asn Gly Thr Ala Asn
   85 90 95
   Lys Ala Asn Lys Val Gly Leu Tyr Phe Tyr Ser Trp Lys Asn Val Asp
   100 105 110
   Lys Glu Asn Asn Phe Thr Leu Lys Glu Gln Thr Thr Ala Asn Asp Tyr
   115 120 125
   Ala Thr Asn Val Asn Ile Gln Leu Met Glu Ser Asn Gly Thr Lys Ala
   130 135 140
   Ile Ser Val Val Gly Lys Glu Thr Glu Asp Phe Met His Thr Asn Asn
   145 150 155 160
   Asn Gly Val Ala Leu Asn Gln Thr Pro Asn Asn Thr His Ile Ser Gly
   165 170 175
   Ser Thr Gln Leu Thr Gly Thr Asn Glu Leu Pro Leu His Phe Ile Ala
   180 185 190
   Gln Tyr Tyr Ala Thr Asn Lys Ala Thr Ala Gly Lys Val Gln Ser Ser
   195 200 205
   Val Asp Phe Gln Ile Ala Tyr Glu
   210 215
(2) INFORMATION FOR SEQ ID NO:3:
   (i) SEQUENCE CHARACTERISTICS:
      (A) LENGTH: 214 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:3:
   Met Glu Gln Phe Ile Met Lys Lys Thr Leu Leu Gly Ser Leu Ile Leu
   1               5                   10                  15
   Leu Ala Phe Ala Gly Asn Val Gln Ala Ala Asp Pro Asn Pro Glu Thr
               20                  25                  30
   Lys Gly Lys Val Thr Phe Tyr Gly Lys Val Val Glu Asn Thr Cys Lys
           35                  40                  45
   Val Lys Ser Gly Asn Arg Asp Met Ser Val Val Leu Asn Asp Val Gly
       50                  55                  60
   Lys Ala His Leu Ser Gln Lys Gly Tyr Thr Ala Met Pro Thr Pro Phe
   65                  70                  75                  80
   Thr Ile Thr Leu Glu Gly Cys Asn Ala Asn Thr Gly Thr Lys Pro Lys
                   85                  90                  95
   Ala Asn Lys Val Gly Val Tyr Phe Tyr Ser Trp Asn Asn Ala Asp Lys
               100                 105                 110
   Glu Asn Ser Tyr Thr Leu Lys Ser Thr Leu Thr Gly Thr Asp Lys Ala
           115                 120                 125
   Asp Asn Val Asn Ile Gln Ile Phe Gln Glu Asn Gly Thr Asp Ala Ile
       130                 135                 140
   Gly Val Ala Asp Lys Thr Ile Asp Asp Phe Thr His Lys Asn Asn Gly
   145                 150                 155                 160
   Ser Thr Asn Ser Asp Lys Pro Thr Lys Asn His Ile Ser Ser Ala Thr
                   165                 170                 175
   Ala Leu Asn Asn Gln Asp Gly Ile Ala Leu His Tyr Ile Ala Gln Tyr
               180                 185                 190
   Tyr Ala Thr Gly Met Ala Ser Ala Gly Lys Gly Pro Thr Ser Val Asp
           195                 200                 205
   Phe Pro Ile Ala Tyr Glu
       210
(2) INFORMATION FOR SEQ ID NO:4:
   (i) SEQUENCE CHARACTERISTICS:
      (A) LENGTH: 9432 base pairs
      (B) TYPE: nucleic acid
      (C) STRANDEDNESS: single
      (D) TOPOLOGY: linear
   (ii) MOLECULE TYPE: DNA (genomic)
   (ix) FEATURE:
      (A) NAME/KEY: CDS
      (B) LOCATION: complement (1882..2532)
   (ix) FEATURE:
      (A) NAME/KEY: CDS
      (B) LOCATION: 2854..3630
   (ix) FEATURE:
      (A) NAME/KEY: CDS
      (B) LOCATION: 4016..6238
   (ix) FEATURE:
      (A) NAME/KEY: CDS
      (B) LOCATION: 6259..6873
   (ix) FEATURE:
      (A) NAME/KEY: CDS
      (B) LOCATION: 6955..8265
   (ix) FEATURE:
      (A) NAME/KEY: CDS
      (B) LOCATION: 8395..9342
   (xi) SEQUENCE DESCRIPTION: SEQ ID NO:4:
   AAGCTTGCAT GCCTGCAGGT CGACTCTAGA GGATCATTCC ATTGTGTTTT ATCTTTTAAT 60
   AAACACCAAG GTGAGGTAGA AATATTCAGT TCATCAAGCA AGGATTTTTG CGTAAAACGA 120
   TCGGCTAATA ATCCAAATAC ATGTTGATTA ACGAAGTTTT TATGATTGCT GAGTAATTCA 180
   GTCAAAGGCG TTTTTTCCCA GCGTTCAATT TCCGCCGTGA TGATCGCATT TTCAGGTAAG 240
   TCAAAAACTG GCGCATTGAA GGCTAAGGGT TCAACATAAA TATCTAAAGG TGCACCAGCG 300
   TAACCTAACA TTCTGCCGAG TTGTCCGTTG CCGAGAACAT AAACGGTTGG GTATAAGGTG 360
   GAGTTTTGCA TAATATTTCT CGTTAAATTT ACGAAAAAAC AACCGCACTT TAAAAGTGCG 420
   GTCAGATCTG AAGATATTTT TATGTGCGTG GATCGGGATT GTCCAGTACA GCACGAGTTT 480
   GGCTTTCACG GAAAGATTGC AAGCGTGAAA GCAATTCTGC ATCCCAACCT GCTAGAATTT 540
   GGGCTGCTAA CAACCCAGCA TTTGCCGCGC CTGCAGAGCC AATCGCTAAT GTTCCGACTG 600
   GAATCCCTTT TGGCATTTGC ACAATTGAAT AAAGGCTATC CACACCACTT AACATAGAAC 660
   TTTTTACTGG CACCCCCAGC ACTGGCACAA GTGTTTTGGC TGCGATCATA CCAGGTAAAT 720
   GTGCCGCACC GCCTGCACCA GCAATAATTA CTTTATAGCC ATTTTTTTGT GCATTTTCGG 780
   CAAATTCGAA AAGTTTATCA GGCGTACGAT GGGCAGAGAC GACTTCCACA TGATAAGGCA 840
   CGTTTAATTC ATCTAAAATC TGAGTTGCCT CTTGCATAGT AGCCCAATCA CTTTTTGACC 900
   CCATCACAAC GGCAATTTGT GCAGTTTTTG ACATGCTATT TTCTCAATTT TCTAATTAAA 960
   AACGTGGTGT AGAATAGCAT AGATTACATA TATCGAGCAA ACGTTTGCTA TTTATGTACG 1020
   TATTAATGGG GATTATTTTA TAATTATTTG ATTTTTAAAT TTTAGTAACT ATACTTGATA 1080
   CCAAATTAAT GGGCGATAGT TTATATGGGA CGAACTGAAA AATTATTAGA TAAGCTCGCA 1140
   CAATCAAAAT CTACATTTAA TTGGAATGAA TTAGTTTCTT TGTTAGCTCA ACAAGGTTAT 1200
   GAAAAGCGAG AAATGGCAGG TTCTCGAGTG AGATTTTATA ATAGAACACT CGAACATATG 1260
   ATTTTGTTAC ACAAGCCTCA TCCTGAAAAT TATATTAAAG GCGGTGTTTT AAAGTCAGTG 1320
   AAAGAATCAT TAAAACAGGT AGGTATTCTA TGAAGTTATT AAATTATAAA GGTTATGTTG 1380
   GCACGATTGA GGCGGATTTA GAAAACAATA TATTATTTGG CAAACTTGCT TACATTCGTG 1440
   ATTTAGTGAC TTACGAAGCA GAGTCATTAT CTGAGTTAGA AAAAGAATTT CATCAATCTG 1500
   TTGATTTATA TTTACAAGAT TGTTTGGAAT TAGGTAAAGA ACCGAATAAG CCTTTTAAAG 1560
   GTGTATTTAA TGTACGAATT GGCGAGGAAT TGCATAGAGA AGCAACGATC ATAGCTGGCG 1620
   ATCGTTCTCT TAATGCTTTT GTGACGGAAG CAATTAAAGA AAAAATTTTT CGTGAAAAAC 1680
   CAAGTTTAAG ATAACAAAAC GTATTTACAT TTTTTTTCAT CACGTAGGCT GGGCGTAAGC 1740
   CCATGTAGAG ACACATAAAA AAGATTTGTA GGCTAGGCGT AAGCTCACGT GGATACATAT 1800
   AAAAAAGATT TGTAGGGTGG GCGTAAGCCC ACGCAGGATA TAACAAACAC GTGGGCTTAG 1860
   ATTGCATTAC ATTAGGAATT ATTCGTAAGC AATTTGGAAA TCAACTGAGG ATTCTACTTT 1920
   ACCAGCTTCC GCTTGAGCTG TTGCATAGTA TCTAGCGATA TAGTGTAATT TCACATTGTT 1980
   TTCACCGTTA ATTGTAGCTT TTCCTGAAAT ATGATTTTTA TTCACAGTTT GTTGTGTTGC 2040
   AACGTCATTT GTATTGCTAT GCGTAAAATC TGTTGTTCCG TTGCCGACAA CTTCAATTGC 2100
   ATCTGTACCA TTAGCATCAA AAAGCTGGAT ATTAACATTC TGTGCAGCAT CATTTCCTGA 2160
   TTTTGTATTT TTTAATGTAT ATTCATTATT TTCATCTGCA TTTTTCCAAG AATAGAAATA 2220
   AGCTCCAACT TTTGTTGCAA CAGGCTTATT ATTAGTAGTA GTAGTAGTAG AACAATTTTC 2280
   TAAATTAATT GTAAATGGTG TTGGCATCGC TGTATCTTTT TTAGTTTTTA AATGATTTTT 2340
   ACCCACATCA TTTAATACTA CGCTCATATT TTTACTATCC GTTTTCACTT TACAAGTATT 2400
   CTCAACAACC TTACCAAAGA AAGTAACTTT ACCAGATGTT TCAGTACTTA CTTGAGGATC 2460
   AGCAGCATTC GTTGCAAATG CCAATAAAAT TAAGCTACCA AGAAGTGTTT TTTTCATAAT 2520
   AAATTGCTCC ATAAAGAGGT TTGTGCCTTA TAAATAAGGC AATAAAGATT AATATAAACC 2580
   GTTTATTAAA ATGCCAAAGG CTTAATAAAC AGCAAACTTT GTTTTCCCAA AAAAAGTAAA 2640
   AAACTCTTCC ATTATATATA TATATATATA TAATTAAAGC CCTTTTTGAA AAATTTCATA 2700
   TTTTTTTGAA TTAATTCGCT GTAGGTTGGG TTTTTGCCCA CATGGAGACA TATAAAAAAG      2760
   ATTTGTAGGG TGGGCGTAAG CCCACGCGGA ACATCATCAA ACAACTGTAA TGTTGTATTA      2820
   GGCACGGTGG GCTTATGCCT CGCCTACGGG GAA ATG AAT AAG GAT AAA TAT GGG      2874
                                        Met Asn Lys Asp Lys Tyr Gly
                                          1               5
   CTT AGC CCA GTT TAT GGA TTT AAT TAT GTT GAA ATG GGG AAA ACA ATG      2922
   Leu Ser Pro Val Tyr Gly Phe Asn Tyr Val Glu Met Gly Lys Thr Met
            10                  15                  20
   TTT AAA AAA ACA CTT TTA TTT TTT ACC GCA CTA TTT TTT GCC GCA CTT      2970
   Phe Lys Lys Thr Leu Leu Phe Phe Thr Ala Leu Phe Phe Ala Ala Leu
        25                  30                  35
   TGT GCA TTT TCA GCC AAT GCA GAT GTG ATT ATC ACT GGC ACC AGA GTG      3018
   Cys Ala Phe Ser Ala Asn Ala Asp Val Ile Ile Thr Gly Thr Arg Val
    40                  45                  50                  55
   ATT TAT CCC GCT GGG CAA AAA AAT GTT ATC GTG AAG TTA GAA AAC AAT      3066
   Ile Tyr Pro Ala Gly Gln Lys Asn Val Ile Val Lys Leu Glu Asn Asn
                    60                  65                  70
   GAT GAT TCG GCA GCA TTG GTG CAA GCC TGG ATT GAT AAT GGC AAT CCA      3114
   Asp Asp Ser Ala Ala Leu Val Gln Ala Trp Ile Asp Asn Gly Asn Pro
                75                  80                  85
   AAT GCC GAT CCA AAA TAC ACC AAA ACC CCT TTT GTG ATT ACC CCG CCT      3162
   Asn Ala Asp Pro Lys Tyr Thr Lys Thr Pro Phe Val Ile Thr Pro Pro
            90                  95                 100
   GTT GCT CGA GTG GAA GCG AAA TCA GGG CAA AGT TTG CGG ATT ACG TTC      3210
   Val Ala Arg Val Glu Ala Lys Ser Gly Gln Ser Leu Arg Ile Thr Phe
       105                 110                 115
   ACA GGC AGC GAG CCT TTA CCT GAT GAT CGC GAA AGC CTC TTT TAT TTT      3258
   Thr Gly Ser Glu Pro Leu Pro Asp Asp Arg Glu Ser Leu Phe Tyr Phe
   120                 125                 130                 135
   AAT TTG TTA GAT ATT CCG CCG AAA CCT GAT GCG GCA TTT CTG GCA AAA      3306
   Asn Leu Leu Asp Ile Pro Pro Lys Pro Asp Ala Ala Phe Leu Ala Lys
                   140                 145                 150
   CAC GGC AGC TTT ATG CAA ATT GCC ATT CGC TCA CGT TTG AAG TTG TTT      3354
   His Gly Ser Phe Met Gln Ile Ala Ile Arg Ser Arg Leu Lys Leu Phe
               155                 160                 165
   TAT CGC CCT GCG AAA CTC TCG ATG GAT TCT CGT GAT GCA ATG AAA AAA      3402
   Tyr Arg Pro Ala Lys Leu Ser Met Asp Ser Arg Asp Ala Met Lys Lys
           170                 175                 180
   GTA GTG TTT AAA GCC ACA CCT GAA GGG GTG TTG GTG GAT AAT CAA ACC      3450
   Val Val Phe Lys Ala Thr Pro Glu Gly Val Leu Val Asp Asn Gln Thr
       185                 190                 195
   CCT TAT TAT ATG AAC TAC ATT GGT TTG TTA CAT CAA AAT AAA CCT GCG      3498
   Pro Tyr Tyr Met Asn Tyr Ile Gly Leu Leu His Gln Asn Lys Pro Ala
   200                 205                 210                 215
   AAA AAT GTC AAA ATG GTT GCC CCT TTT TCT CAA GCG GTA TTT GAA GCC      3546
   Lys Asn Val Lys Met Val Ala Pro Phe Ser Gln Ala Val Phe Glu Ala
                   220                 225                 230
   AAA GGC GTG CGT TCT GGC GAT AAA TTG AAA TGG GTA TTG GTT AAT GAT      3594
   Lys Gly Val Arg Ser Gly Asp Lys Leu Lys Trp Val Leu Val Asn Asp
               235                 240                 245
   TAC GGT GCC GAC CAA GAA GGC GAA GCC ATC GCT CAA TAATAGCGAA           3640
   Tyr Gly Ala Asp Gln Glu Gly Glu Ala Ile Ala Gln
           250                 255
   CTAGTGTAGG GTGGGCTTTA GACCACCGAT TAACCATAAC AAAGGTGGGC TGAAGCCCAC    3700
   CCTACAACCA CAAAGAACGA TTAATCTGTG AAAACAAAAA TTTTTCCCTT AAATAAAATT    3760
   GCGTTTGCTT GTTCACTGCT ATTGGCAAAT CCTTTAGCGT GGGCGGGAGA TCAATTTGAT    3820
   GCCTCTCTTT GGGGAGATGG TTCGGTGTTG GGCGTTGATT TTGCCCGATT TAATGTAAAA    3880
   AATGCCGTGT TACCAGGGCG TTATGAAGCT CAAATCTATG TGAAATTTGA AGAAAAAGGC    3940
   GTAAGCGATA TTATTTTTGC TGATAATCCT GCCACAGGTC GGACAGAATT ATGCTTTACG 4000
   CCTAAACTTC AAGAA ATG CTG GAT TTG ATG GAT GAA GCC ATT GTG AAA TCG 4051
   Met Leu Asp Leu Met Asp Glu Ala Ile Val Lys Ser
   1 5 10
   CCC AAT TCA GAA GAT GAC ACT TGT GTC TTT GCT TCT GAT GCT ATT CCT 4099
   Pro Asn Ser Glu Asp Asp Thr Cys Val Phe Ala Ser Asp Ala Ile Pro
   15 20 25
   AAA GGC ACG TTT GAA TAT CAA AGC GGC GAA ATG AAA TTG AAA CTT GAG 4147
   Lys Gly Thr Phe Glu Tyr Gln Ser Gly Glu Met Lys Leu Lys Leu Glu
   30 35 40
   CTC CCT CAA GCT CTC ACT ATT CGC CGA CCA AGA GGC TAT ATT GCG CCA 4195
   Leu Pro Gln Ala Leu Thr Ile Arg Arg Pro Arg Gly Tyr Ile Ala Pro
   45 50 55 60
   TCT CGC TGG CAA ACT GGC ACC AAT GCC GCT TTT GCA AAT TAC GAT ATC 4243
   Ser Arg Trp Gln Thr Gly Thr Asn Ala Ala Phe Ala Asn Tyr Asp Ile
   65 70 75
   AAC TAT TAT CGT TCT GGT AAT CCC GAA GTA AAA TCC GAA AGT TTG TAT 4291
   Asn Tyr Tyr Arg Ser Gly Asn Pro Glu Val Lys Ser Glu Ser Leu Tyr
   80 85 90
   GTG GGC TTG CGT AGT GGC GTA AAT TTT GGC AAC TGG GCA TTG CGT CAT 4339
   Val Gly Leu Arg Ser Gly Val Asn Phe Gly Asn Trp Ala Leu Arg His
   95 100 105
   AGC GGC AGT TTT AGC CGT TTT GAA AAC CAA AGT AGC TCG GGT TTT ACT 4387
   Ser Gly Ser Phe Ser Arg Phe Glu Asn Gln Ser Ser Ser Gly Phe Thr
   110 115 120
   GAT AAG GGC AAA AAT CAT TAC GAA CGT GGC GAT ACC TAT TTA CAA CGA 4435
   Asp Lys Gly Lys Asn His Tyr Glu Arg Gly Asp Thr Tyr Leu Gln Arg
   125 130 135 140
   GAT TTC GCC CTG CTT CGT GGC AAT GTC ACT GTT GGG GAT TTT TTC AGC 4483
   Asp Phe Ala Leu Leu Arg Gly Asn Val Thr Val Gly Asp Phe Phe Ser
   145 150 155
   ACT GCC CGC ATT GGC GAA AAT TTT GGT ATG CGT GGT TTG CGT ATT GCC 4531
   Thr Ala Arg Ile Gly Glu Asn Phe Gly Met Arg Gly Leu Arg Ile Ala
   160 165 170
   TCT GAT GAT AGA ATG CTT GCC CCA TCA CAA CGT GGT TTT GCC CCA GTG 4579
   Ser Asp Asp Arg Met Leu Ala Pro Ser Gln Arg Gly Phe Ala Pro Val
   175 180 185
   GTG CGT GGC GTG GCA AAC ACA AAC GCC AAA GTC AGC ATC AAA CAA AAT 4627
   Val Arg Gly Val Ala Asn Thr Asn Ala Lys Val Ser Ile Lys Gln Asn
   190 195 200
   GGC TAT ACG ATT TAT CAA ATC ACC GTT CCC GCA GGG CCT TTC GTG ATT 4675
   Gly Tyr Thr Ile Tyr Gln Ile Thr Val Pro Ala Gly Pro Phe Val Ile
   205 210 215 220
   AAC GAT TTG TAT GCC AGC GGT TAT AGC GGC GAT TTA ACG GTG GAA ATC 4723
   Asn Asp Leu Tyr Ala Ser Gly Tyr Ser Gly Asp Leu Thr Val Glu Ile
   225 230 235
   CAA GAA AGT GAT GGT AAA GTG CGG TCA TTT ATT GTG CCG TTT TCT AAT 4771
   Gln Glu Ser Asp Gly Lys Val Arg Ser Phe Ile Val Pro Phe Ser Asn
   240 245 250
   CTT GCC CCG TTA ATG CGT GTG GGG CAT TTG CGT TAT CAA TTA GCT GGC 4819
   Leu Ala Pro Leu Met Arg Val Gly His Leu Arg Tyr Gln Leu Ala Gly
   255 260 265
   GGA CGT TAT CGA ATT GAC AGC CGC ACC TTT GAT GAA CGT GTG TTA CAA 4867
   Gly Arg Tyr Arg Ile Asp Ser Arg Thr Phe Asp Glu Arg Val Leu Gln
   270 275 280
   GGC GTG TTG CAA TAT GGT TTA ACT AAT CAT CTC ACG CTG AAT TCA AGC 4915
   Gly Val Leu Gln Tyr Gly Leu Thr Asn His Leu Thr Leu Asn Ser Ser
   285 290 295 300
   CTG CTT TAT ACA CGT CAT TAT CGT GCA GGG CTG TTT GGT TTT GGT TTA 4963
   Leu Leu Tyr Thr Arg His Tyr Arg Ala Gly Leu Phe Gly Phe Gly Leu
   305 310 315
   AAT ACG CCG ATT GGG GCG TTT TCT GCT GAT GCC ACT TGG TCG CAC GCT 5011
   Asn Thr Pro Ile Gly Ala Phe Ser Ala Asp Ala Thr Trp Ser His Ala
   320 325 330
   GAA TTT CCG CTA AAA CAT GTG AGC AAA AAC GGC TAC AGC TTG CAC GGC 5059
   Glu Phe Pro Leu Lys His Val Ser Lys Asn Gly Tyr Ser Leu His Gly
   335 340 345
   AGT TAT AGT ATT AAC TTC AAT GAA AGT GGC ACC AAT ATC ACG TTG GCA 5107
   Ser Tyr Ser Ile Asn Phe Asn Glu Ser Gly Thr Asn Ile Thr Leu Ala
   350 355 360
   GCC TAT CGC TAT TCT TCA CGG GAT TTT TAC ACC TTA AGC GAC ACC ATT 5155
   Ala Tyr Arg Tyr Ser Ser Arg Asp Phe Tyr Thr Leu Ser Asp Thr Ile
   365 370 375 380
   GGT CTT AAC CGC ACT TTC AGA CAA TTT AGC GGT GCG TAT TTG CCT GAA 5203
   Gly Leu Asn Arg Thr Phe Arg Gln Phe Ser Gly Ala Tyr Leu Pro Glu
   385 390 395
   ATT TAC CGC CCA AAA AAT CAG TTT CAA GTG AGT TTA AGC CAA AGT CTG 5251
   Ile Tyr Arg Pro Lys Asn Gln Phe Gln Val Ser Leu Ser Gln Ser Leu
   400 405 410
   GGG AAT TGG GGA AAT CTC TAT CTT TCA GGA CAA ACC TAT AAT TAT TGG 5299
   Gly Asn Trp Gly Asn Leu Tyr Leu Ser Gly Gln Thr Tyr Asn Tyr Trp
   415 420 425
   GAA AAA CGT GGC ACG AAT ACG CAA TAT CAA GTT GCC TAT TCA AAC AGC 5347
   Glu Lys Arg Gly Thr Asn Thr Gln Tyr Gln Val Ala Tyr Ser Asn Ser
   430 435 440
   TTC CAC ATT CTT AAT TAC TCT GTA AAC CTC TCA CAG AGT ATT GAT AAA 5395
   Phe His Ile Leu Asn Tyr Ser Val Asn Leu Ser Gln Ser Ile Asp Lys
   445 450 455 460
   GAA ACG GGC AAA CGT GAC AAC AGC ATT TAT TTA AGT CTC AGC CTG CCA 5443
   Glu Thr Gly Lys Arg Asp Asn Ser Ile Tyr Leu Ser Leu Ser Leu Pro
   465 470 475
   TTA GGC GAT AAC CAT TCT GCA GAT AGT AGT TAT TCT CGC AGT GGT AAC 5491
   Leu Gly Asp Asn His Ser Ala Asp Ser Ser Tyr Ser Arg Ser Gly Asn
   480 485 490
   GAT ATT AAC CAA CGA CTT GGC GTA AAT GGC TCT TTT GGT GAA CGT CAT 5539
   Asp Ile Asn Gln Arg Leu Gly Val Asn Gly Ser Phe Gly Glu Arg His
   495 500 505
   CAA TGG AGT TAT GGT ATT AAC GCT TCA CGC AAT AAT CAA GGC TAT CGC 5587
   Gln Trp Ser Tyr Gly Ile Asn Ala Ser Arg Asn Asn Gln Gly Tyr Arg
   510 515 520
   AGT TAT GAC GGT AAT CTT TCG CAT AAC AAT AGC ATT GGT AGT TAC CGT 5635
   Ser Tyr Asp Gly Asn Leu Ser His Asn Asn Ser Ile Gly Ser Tyr Arg
   525 530 535 540
   GCT TCT TAT TCA CGT GAT AGC CTC AAA AAT CGC TCC ATC TCA CTG GGC 5683
   Ala Ser Tyr Ser Arg Asp Ser Leu Lys Asn Arg Ser Ile Ser Leu Gly
   545 550 555
   GCA AGC GGT GCT GTC GTG GCG CAC AAA CAC GGT ATT ACC TTA AGC CAA 5731
   Ala Ser Gly Ala Val Val Ala His Lys His Gly Ile Thr Leu Ser Gln
   560 565 570
   CCT GTT GGC GAA AGT TTT GCC ATT ATT CAC GCC AAA GAT GCC GCA GGA 5779
   Pro Val Gly Glu Ser Phe Ala Ile Ile His Ala Lys Asp Ala Ala Gly
   575 580 585
   GCA AAA GTG GAA TCA GGT GCC AAT GTG AGC CTT GAT TAT TTC GGC AAT 5827
   Ala Lys Val Glu Ser Gly Ala Asn Val Ser Leu Asp Tyr Phe Gly Asn
   590 595 600
   GCG GTT ATG CCT TAC ACC AGC CCG TAT GAA ATC AAT TAT ATC GGT ATC 5875
   Ala Val Met Pro Tyr Thr Ser Pro Tyr Glu Ile Asn Tyr Ile Gly Ile
   605 610 615 620
   AAT CCA TCT GAT GCG GAG GCG AAT GTG GAA TTT GAA GCC ACT GAA CGC 5923
   Asn Pro Ser Asp Ala Glu Ala Asn Val Glu Phe Glu Ala Thr Glu Arg
   625 630 635
   CAA ATC ATT CCT CGT GCA AAT TCA ATT AGC TTA GTA GAT TTC CGC ACG 5971
   Gln Ile Ile Pro Arg Ala Asn Ser Ile Ser Leu Val Asp Phe Arg Thr
   640 645 650
   GGC AAA AAT ACA ATG GTG TTA TTT AAC CTC ACT TTG CCA AAT GGC GAG 6019
   Gly Lys Asn Thr Met Val Leu Phe Asn Leu Thr Leu Pro Asn Gly Glu
   655 660 665
   CCA GTG CCA ATG GCA TCC ACC GCA CAA GAT AGC GAA GGG GCA TTT GTG 6067
   Pro Val Pro Met Ala Ser Thr Ala Gln Asp Ser Glu Gly Ala Phe Val
   670 675 680
   GGC GAT GTG GTG CAA GGT GGT GTG CTT TTC GCT AAT AAA CTT ACC CAG 6115
   Gly Asp Val Val Gln Gly Gly Val Leu Phe Ala Asn Lys Leu Thr Gln
   685 690 695 700
   CCA AAA GGC GAG TTA ATC GTC AAA TGG GGT GAG CGA GAA AGC GAA CAA 6163
   Pro Lys Gly Glu Leu Ile Val Lys Trp Gly Glu Arg Glu Ser Glu Gln
   705 710 715
   TGC CGT TTC CAA TAT CAA GTT GAT TTG GAT AAC GCA CAA ATA CAA AGT 6211
   Cys Arg Phe Gln Tyr Gln Val Asp Leu Asp Asn Ala Gln Ile Gln Ser
   720 725 730
   CAC GAT ATT CAA TGC AAA ACC GCA AAA TAAATAATTG AAGAGGATTT ATG 6261
   His Asp Ile Gln Cys Lys Thr Ala Lys Met
   735 740 1
   CAA AAA ACA CCC AAA AAA TTA ACC GCG CTT TTC CAT CAA AAA TCC ACT 6309
   Gln Lys Thr Pro Lys Lys Leu Thr Ala Leu Phe His Gln Lys Ser Thr
   5 10 15
   GCT ACT TGT AGT GGA GCA AAT TAT AGT GGA GCA AAT TAT AGT GGC TCA 6357
   Ala Thr Cys Ser Gly Ala Asn Tyr Ser Gly Ala Asn Tyr Ser Gly Ser
   20 25 30
   AAA TGC TTT AGG TTT CAT CGT CTG GCT CTG CTT GCT TGC GTG GCT CTG 6405
   Lys Cys Phe Arg Phe His Arg Leu Ala Leu Leu Ala Cys Val Ala Leu
   35 40 45
   CTT GAT TGC ATT GTG GCA CTG CCT GCT TAT GCT TAC GAT GGC AGA GTG       6453
   Leu Asp Cys Ile Val Ala Leu Pro Ala Tyr Ala Tyr Asp Gly Arg Val
    50                  55                  60                  65
   ACC TTT CAA GGG GAG ATT TTA AGT GAT GGC ACT TGT AAA ATT GAA ACA       6501
   Thr Phe Gln Gly Glu Ile Leu Ser Asp Gly Thr Cys Lys Ile Glu Thr
                    70                  75                  80
   GAC AGC CAA AAT CGC ACG GTT ACC CTG CCA ACA GTG GGA AAA GCT AAT       6549
   Asp Ser Gln Asn Arg Thr Val Thr Leu Pro Thr Val Gly Lys Ala Asn
                85                  90                  95
   TTA AGC CAC GCA GGG CAA ACC GCC GCC CCT GTG CCT TTT TCC ATC ACG       6597
   Leu Ser His Ala Gly Gln Thr Ala Ala Pro Val Pro Phe Ser Ile Thr
           100                 105                 110
   TTA AAA GAA TGC AAT GCA GAT GAT GCT ATG AAA GCT AAT CTG CTA TTT       6645
   Leu Lys Glu Cys Asn Ala Asp Asp Ala Met Lys Ala Asn Leu Leu Phe
       115                 120                 125
   AAA GGG GGA GAC AAC ACA ACA GGG CAA TCT TAT CTT TCC AAT AAG GCA       6693
   Lys Gly Gly Asp Asn Thr Thr Gly Gln Ser Tyr Leu Ser Asn Lys Ala
   130                 135                 140                 145
   GGC AAC GGC AAA GCC ACC AAC GTG GGC ATT CAA ATT GTC AAA GCC GAT       6741
   Gly Asn Gly Lys Ala Thr Asn Val Gly Ile Gln Ile Val Lys Ala Asp
                   150                 155                 160
   GGC ATA GGC ACG CCT ATC AAG GTG GAC GGC ACC GAA GCC AAC AGC GAA       6789
   Gly Ile Gly Thr Pro Ile Lys Val Asp Gly Thr Glu Ala Asn Ser Glu
               165                 170                 175
   AAA GCC CCC GAC ACA GGT AAA GCG CAA AAC GGC ACA GTT ATT CAA CCC       6837
   Lys Ala Pro Asp Thr Gly Lys Ala Gln Asn Gly Thr Val Ile Gln Pro
           180                 185                 190
   CGT TTT GGC TAC TTT GGC TCG TTA TTA CGC CAC AGG TGAAGCCACC            6883
   Arg Phe Gly Tyr Phe Gly Ser Leu Leu Arg His Arg
       195                 200                 205
   GCAGGCGACG TTGAAGCCAC TGCAACTTTT GAAGTGCAGT ATAACTAAAA TATTTATTAT    6943
   CCAGTGAAAA A ATG AAT AAG AAA TCG TAT ATA AAT CAT TAC TTA ACT TTA     6993
                Met Asn Lys Lys Ser Tyr Ile Asn His Tyr Leu Thr Leu
                 1               5                  10
   TTT AAA GTT ACT ACT TTA CTA TTT ACT CTT TCA AGT AAT CCT GTA TGG      7041
   Phe Lys Val Thr Thr Leu Leu Phe Thr Leu Ser Ser Asn Pro Val Trp
       15                  20                  25
   GCA AAT ATA AAA ACA GTT CAG GGA ACA ACT AGT GGT TTT CCA CTT CTA      7089
   Ala Asn Ile Lys Thr Val Gln Gly Thr Thr Ser Gly Phe Pro Leu Leu
   30                  35                  40                  45
   ACA AGA ACT TTC ACA TTT AAT GGC AAT TTG CAA TGG AAT GTG AGT GCT      7137
   Thr Arg Thr Phe Thr Phe Asn Gly Asn Leu Gln Trp Asn Val Ser Ala
                   50                  55                  60
   CTA CAA CCA GCT TAT ATT GTT TCC TCT CAA GCA AGA GAT AAT CTT GAT      7185
   Leu Gln Pro Ala Tyr Ile Val Ser Ser Gln Ala Arg Asp Asn Leu Asp
               65                  70                  75
   ACA GTA CAT ATT CAA TCT TCT GAA ATT AAT GCT CCA ACA AAT TCA TTA      7233
   Thr Val His Ile Gln Ser Ser Glu Ile Asn Ala Pro Thr Asn Ser Leu
           80                  85                  90
   GCT CCA TTT AAT AAT TGG ATT AAT ACG AAA TCA GCA GTA GAG CTA GGT      7281
   Ala Pro Phe Asn Asn Trp Ile Asn Thr Lys Ser Ala Val Glu Leu Gly
       95                  100                 105
   TAT AGC TTT GCG GGC ATT ACT TGT ACT AGT AAT CCT TGC CCA ACA ATG      7329
   Tyr Ser Phe Ala Gly Ile Thr Cys Thr Ser Asn Pro Cys Pro Thr Met
   110                 115                 120                 125
   AAA TTA CCA TTA TTA TTT CAT CCT GAT CTT ACT AAT TTA ACT CCA CCT      7377
   Lys Leu Pro Leu Leu Phe His Pro Asp Leu Thr Asn Leu Thr Pro Pro
                   130                 135                 140
   GGA AAG AAA AAT TCT GAT GGA GGG GAG ATT TTT AAA TTA CAT AAT GAA      7425
   Gly Lys Lys Asn Ser Asp Gly Gly Glu Ile Phe Lys Leu His Asn Glu
               145                 150                 155
   TCT AAT TTA GGC GTC TCT TTT CAA ATT GGA GTA AAA ACG AAT ACT TCT 7473
   Ser Asn Leu Gly Val Ser Phe Gln Ile Gly Val Lys Thr Asn Thr Ser
   160 165 170
   CTA GAT TGG GTT AAT GCT AAG AAT AAT TTT AGC TCT CTA AAA GTT TTA 7521
   Leu Asp Trp Val Asn Ala Lys Asn Asn Phe Ser Ser Leu Lys Val Leu
   175 180 185
   ATG GTG CCT TTT AAT TCT AGC GAT AAA ATA TCT TTG CAT TTA CGT GCT 7569
   Met Val Pro Phe Asn Ser Ser Asp Lys Ile Ser Leu His Leu Arg Ala
   190 195 200 205
   AAA TTT CAT TTA TTA ACA GAT TTT TCA TCG CTA AAT AAT GAT ATT ACT 7617
   Lys Phe His Leu Leu Thr Asp Phe Ser Ser Leu Asn Asn Asp Ile Thr
   210 215 220
   ATT GAC CCT ATG AAT ACT AGT ATA GGC AAA ATT AAT CTT GAA ACG TGG 7665
   Ile Asp Pro Met Asn Thr Ser Ile Gly Lys Ile Asn Leu Glu Thr Trp
   225 230 235
   CGT GGC TCA ACA GGC AAT TTT TCT GTT AAA TAT GTA GGT GAG GAT AAG 7713
   Arg Gly Ser Thr Gly Asn Phe Ser Val Lys Tyr Val Gly Glu Asp Lys
   240 245 250
   GGA GAT ATA TCT ATT TTC TTT AAT ACA CCT AAA ATT ATT CTA AAA AAA 7761
   Gly Asp Ile Ser Ile Phe Phe Asn Thr Pro Lys Ile Ile Leu Lys Lys
   255 260 265
   CAA CAA CGC CGA TGT ACT CTG AAT AAT GCT CCA GTG AGC CCA AAT CCA 7809
   Gln Gln Arg Arg Cys Thr Leu Asn Asn Ala Pro Val Ser Pro Asn Pro
   270 275 280 285
   GTT AAA TTA CGA GCG GTA AAA AAA CGT GAA TTG GAG GCA CAA AGT GAA 7857
   Val Lys Leu Arg Ala Val Lys Lys Arg Glu Leu Glu Ala Gln Ser Glu
   290 295 300
   ATG GAA GGT GGG ACA TTT CAG TTA AGA GTA AAT TGT GAC AAT ACC ACT 7905
   Met Glu Gly Gly Thr Phe Gln Leu Arg Val Asn Cys Asp Asn Thr Thr
   305 310 315
   TAT AAT AAA GCC AAC GGC AAA TGG TTA TTT CCT GTA GTG AAA GTT ACT        7953
   Tyr Asn Lys Ala Asn Gly Lys Trp Leu Phe Pro Val Val Lys Val Thr
           320                 325                 330
   TTT ACG GAC GAA GAT GGT ACA ACG AAT AAT GGA ACA AAT GAC TTA CTT        8001
   Phe Thr Asp Glu Asp Gly Thr Thr Asn Asn Gly Thr Asn Asp Leu Leu
       335                 340                 345
   CGC ACC CAA ACA GGC AGC GGA CAA GCC ACA GGC GTT AGC TTA AGA ATC        8049
   Arg Thr Gln Thr Gly Ser Gly Gln Ala Thr Gly Val Ser Leu Arg Ile
   350                 355                 360                 365
   AAA CGA GAA AAT GGT ACA GAA ACC GTA AAA TAC GGT GCT GAT TCT GCT        8097
   Lys Arg Glu Asn Gly Thr Glu Thr Val Lys Tyr Gly Ala Asp Ser Ala
                   370                 375                 380
   CAA ATG GGG AAT GCT GGA CAA TTT GAA TTA CGA AAA CAA CCA TCC CCT        8145
   Gln Met Gly Asn Ala Gly Gln Phe Glu Leu Arg Lys Gln Pro Ser Pro
               385                 390                 395
   GCT GGT GGA GAT CAA TAT GCT GAA GAA ACT TTC AAA GTC TAT TAC GTA        8193
   Ala Gly Gly Asp Gln Tyr Ala Glu Glu Thr Phe Lys Val Tyr Tyr Val
           400                 405                 410
   AAA GAC TCA ACA AGA GGC ACC TTA ATC GAA GGA AAA GTC AAA GCC GCC        8241
   Lys Asp Ser Thr Arg Gly Thr Leu Ile Glu Gly Lys Val Lys Ala Ala
       415                 420                 425
   GCC ACT TTC ACA ATG TCA TAT CAA TAATAATGTC GGGTGGGAAT ATAAAGGCTG       8295
   Ala Thr Phe Thr Met Ser Tyr Gln
   430                 435
   AAGGTTTAAA CTTCAGTCTT TTTTTATAGG AAAATACCAT TGCAACTTTA AGGATAAAAT      8355
   TTTATCCTAA GCACAATTTT TATAAGAATA GGTCAAATT ATG TTA GCC AAA GCA         8409
                                              Met Leu Ala Lys Ala
                                              1               5
   AAA TAT AGA AAA GAT TAC AAA CAA CCA GAT TTT ACG GTC ACA GAC ATT        8457
   Lys Tyr Arg Lys Asp Tyr Lys Gln Pro Asp Phe Thr Val Thr Asp Ile
                   10                  15                  20
   TAT TTA GAT TTT CAA CTT GAT CCT AAA AAT ACT GTG GTG ACT GCA ACC      8505
   Tyr Leu Asp Phe Gln Leu Asp Pro Lys Asn Thr Val Val Thr Ala Thr
               25                  30                  35
   ACA AAA TTC CAA CGC TTA AAT AAT GAA GCG ACG TCT TTA CGT TTA GAC      8553
   Thr Lys Phe Gln Arg Leu Asn Asn Glu Ala Thr Ser Leu Arg Leu Asp
           40                  45                  50
   GGG CAT AGC TTC CAG TTT TCT TCT ATT AAA TTT AAT GGC GAG CCA TTT      8601
   Gly His Ser Phe Gln Phe Ser Ser Ile Lys Phe Asn Gly Glu Pro Phe
       55                  60                  65
   TCT GAT TAT CAA CAA GAT GGC GAG AGT TTA ACG CTC GAT TTA AAA GAC      8649
   Ser Asp Tyr Gln Gln Asp Gly Glu Ser Leu Thr Leu Asp Leu Lys Asp
   70                  75                  80                  85
   AAA AGT GCG GAT GAA TTT GAG CTT GAA ATT GTG ACG TTC CTT GTG CCA      8697
   Lys Ser Ala Asp Glu Phe Glu Leu Glu Ile Val Thr Phe Leu Val Pro
                   90                  95                  100
   GCC GAA AAT ACG TCA TTA CAA GGG CTA TAT CAG TCT GGC GAA GGT ATT      8745
   Ala Glu Asn Thr Ser Leu Gln Gly Leu Tyr Gln Ser Gly Glu Gly Ile
               105                 110                 115
   TGT ACG CAA TGT GAG GCG GAA GGT TTC CGT CAA ATC ACT TAT ATG CTT      8793
   Cys Thr Gln Cys Glu Ala Glu Gly Phe Arg Gln Ile Thr Tyr Met Leu
           120                 125                 130
   GAT CGT CCT GAT GTG CTG GCG CGT TAT ATA ATC AAA ATT ACG GCA GAT      8841
   Asp Arg Pro Asp Val Leu Ala Arg Tyr Ile Ile Lys Ile Thr Ala Asp
       135                 140                 145
   AAA ACC AAA TAT CCA TTC TTA CTG TCG AAT GGT AAT CGC ATT GCA AGT      8889
   Lys Thr Lys Tyr Pro Phe Leu Leu Ser Asn Gly Asn Arg Ile Ala Ser
   150                 155                 160                 165
   GGC GAA TTA GAA GAT GGT CGC CAT TGG GTG GAA TGG AAT GAT CCT TTC      8937
   Gly Glu Leu Glu Asp Gly Arg His Trp Val Glu Trp Asn Asp Pro Phe
                   170                 175                 180
   CCA AAA CCA AGC TAT TTA TTT GCT TTA GTG GCG GGA GAT TNN GGT TTA     8985
   Pro Lys Pro Ser Tyr Leu Phe Ala Leu Val Ala Gly Asp Xaa Gly Leu
               185                 190                 195
   TTA CAA GAT AAN TTT ATT ACT AAA AGT GGT CGT GAA GTG GCT TTA GAG     9033
   Leu Gln Asp Xaa Phe Ile Thr Lys Ser Gly Arg Glu Val Ala Leu Glu
           200                 205                 210
   CTT TAT GTG GAT CGC GGT AAT CTT AAC CGT GCA ACT GGG GCA ATG GAA     9081
   Leu Tyr Val Asp Arg Gly Asn Leu Asn Arg Ala Thr Gly Ala Met Glu
       215                 220                 225
   AGT CTG AAA AAA GCG ATG AAA TGG GAT GAA GAT CGC TTT ATT TTA GAA     9129
   Ser Leu Lys Lys Ala Met Lys Trp Asp Glu Asp Arg Phe Ile Leu Glu
   230                 235                 240                 245
   TTT TAC CTA GAT ATT TAT ATG ATC GCG GCC GCC GAT TCC TCC AAT ATG     9177
   Phe Tyr Leu Asp Ile Tyr Met Ile Ala Ala Ala Asp Ser Ser Asn Met
                   250                 255                 260
   GGC GCA ATG GAA AAT AAA GGA TTA AAT ATC TTT AAC TCT AAA TTG GTG     9225
   Gly Ala Met Glu Asn Lys Gly Leu Asn Ile Phe Asn Ser Lys Leu Val
               265                 270                 275
   TTG GCA AAT CCA CAA ACG GCA ACA GAT GAA GAT TAT CTT GTC ATT GAA     9273
   Leu Ala Asn Pro Gln Thr Ala Thr Asp Glu Asp Tyr Leu Val Ile Glu
           280                 285                 290
   AGT GTG ATT GCA CAC GAA TAT TCC CAT AAC TGG ACG GGA AAC CGT GTA     9321
   Ser Val Ile Ala His Glu Tyr Ser His Asn Trp Thr Gly Asn Arg Val
       295                 300                 305
   ACC CGC CGA GAT GGG TTC AAC TAGGTTTGAA GAAGGTTAAC GGCTTCCGGG        9372
   Thr Arg Arg Asp Gly Phe Asn
   310                 315
   AACAAGATTT CTCAGATCAG TTCTCCGGGC CGGAACCGAT TAATAAGGGA AAATTTTCCG   9432
(2) INFORMATION FOR SEQ ID NO:5:
   (i) SEQUENCE CHARACTERISTICS:
      (A) LENGTH: 217 amino acids
      (B) TYPE: amino acid
      (D) TOPOLOGY: linear
   (ii) MOLECULE TYPE: protein
   (xi) SEQUENCE DESCRIPTION: SEQ ID NO:5:
   Met Glu Gln Phe Ile Met Lys Lys Thr Leu Leu Gly Ser Leu Ile Leu
   1 5 10 15
   Leu Ala Phe Ala Thr Asn Ala Ala Asp Pro Gln Val Ser Thr Glu Thr
   20 25 30
   Ser Gly Lys Val Thr Phe Phe Gly Lys Val Val Glu Asn Thr Cys Lys
   35 40 45
   Val Lys Thr Asp Ser Lys Asn Met Ser Val Val Leu Asn Asp Val Gly
   50 55 60
   Lys Asn His Leu Lys Thr Lys Lys Asp Thr Ala Met Pro Thr Pro Phe
   65 70 75 80
   Thr Ile Asn Leu Glu Asn Cys Ser Thr Thr Thr Thr Thr Asn Asn Lys
   85 90 95
   Pro Val Ala Thr Lys Val Gly Ala Tyr Phe Tyr Ser Trp Lys Asn Ala
   100 105 110
   Asp Glu Asn Asn Glu Tyr Thr Leu Lys Asn Thr Lys Ser Gly Asn Asp
   115 120 125
   Ala Ala Gln Asn Val Asn Ile Gln Leu Phe Asp Ala Asn Gly Thr Asp
   130 135 140
   Ala Ile Glu Val Val Gly Asn Gly Thr Thr Asp Phe Thr His Ser Asn
   145 150 155 160
   Thr Asn Asp Val Ala Thr Gln Gln Thr Val Asn Lys Asn His Ile Ser
   165 170 175
   Gly Lys Ala Thr Ile Asn Gly Glu Asn Asn Val Lys Leu His Tyr Ile
   180 185 190
   Ala Arg Tyr Tyr Ala Thr Ala Gln Ala Glu Ala Gly Lys Val Glu Ser
   195 200 205
   Ser Val Asp Phe Gln Ile Ala Tyr Glu
   210 215
(2) INFORMATION FOR SEQ ID NO:6:
   (i) SEQUENCE CHARACTERISTICS:
      (A) LENGTH: 259 amino acids
      (B) TYPE: amino acid
      (D) TOPOLOGY: linear
   (ii) MOLECULE TYPE: protein
   (xi) SEQUENCE DESCRIPTION: SEQ ID NO:6:
   Met Asn Lys Asp Lys Tyr Gly Leu Ser Pro Val Tyr Gly Phe Asn Tyr
    1               5                  10                  15
   Val Glu Met Gly Lys Thr Met Phe Lys Lys Thr Leu Leu Phe Phe Thr
               20                  25                  30
   Ala Leu Phe Phe Ala Ala Leu Cys Ala Phe Ser Ala Asn Ala Asp Val
           35                  40                  45
   Ile Ile Thr Gly Thr Arg Val Ile Tyr Pro Ala Gly Gln Lys Asn Val
       50                  55                  60
   Ile Val Lys Leu Glu Asn Asn Asp Asp Ser Ala Ala Leu Val Gln Ala
   65                  70                  75                  80
   Trp Ile Asp Asn Gly Asn Pro Asn Ala Asp Pro Lys Tyr Thr Lys Thr
                   85                  90                  95
   Pro Phe Val Ile Thr Pro Pro Val Ala Arg Val Glu Ala Lys Ser Gly
   100 105 110
   Gln Ser Leu Arg Ile Thr Phe Thr Gly Ser Glu Pro Leu Pro Asp Asp
   115 120 125
   Arg Glu Ser Leu Phe Tyr Phe Asn Leu Leu Asp Ile Pro Pro Lys Pro
   130 135 140
   Asp Ala Ala Phe Leu Ala Lys His Gly Ser Phe Met Gln Ile Ala Ile
   145 150 155 160
   Arg Ser Arg Leu Lys Leu Phe Tyr Arg Pro Ala Lys Leu Ser Met Asp
   165 170 175
   Ser Arg Asp Ala Met Lys Lys Val Val Phe Lys Ala Thr Pro Glu Gly
   180 185 190
   Val Leu Val Asp Asn Gln Thr Pro Tyr Tyr Met Asn Tyr Ile Gly Leu
   195 200 205
   Leu His Gln Asn Lys Pro Ala Lys Asn Val Lys Met Val Ala Pro Phe
   210 215 220
   Ser Gln Ala Val Phe Glu Ala Lys Gly Val Arg Ser Gly Asp Lys Leu
   225 230 235 240
   Lys Trp Val Leu Val Asn Asp Tyr Gly Ala Asp Gln Glu Gly Glu Ala
   245 250 255
   Ile Ala Gln
(2) INFORMATION FOR SEQ ID NO:7:
   (i) SEQUENCE CHARACTERISTICS:
      (A) LENGTH: 741 amino acids
      (B) TYPE: amino acid
      (D) TOPOLOGY: linear
   (ii) MOLECULE TYPE: protein
   (xi) SEQUENCE DESCRIPTION: SEQ ID NO:7:
   Met Leu Asp Leu Met Asp Glu Ala Ile Val Lys Ser Pro Asn Ser Glu
   1 5 10 15
   Asp Asp Thr Cys Val Phe Ala Ser Asp Ala Ile Pro Lys Gly Thr Phe
   20 25 30
   Glu Tyr Gln Ser Gly Glu Met Lys Leu Lys Leu Glu Leu Pro Gln Ala
   35 40 45
   Leu Thr Ile Arg Arg Pro Arg Gly Tyr Ile Ala Pro Ser Arg Trp Gln
   50 55 60
   Thr Gly Thr Asn Ala Ala Phe Ala Asn Tyr Asp Ile Asn Tyr Tyr Arg
   65 70 75 80
   Ser Gly Asn Pro Glu Val Lys Ser Glu Ser Leu Tyr Val Gly Leu Arg
   85 90 95
   Ser Gly Val Asn Phe Gly Asn Trp Ala Leu Arg His Ser Gly Ser Phe
   100 105 110
   Ser Arg Phe Glu Asn Gln Ser Ser Ser Gly Phe Thr Asp Lys Gly Lys
   115 120 125
   Asn His Tyr Glu Arg Gly Asp Thr Tyr Leu Gln Arg Asp Phe Ala Leu
   130 135 140
   Leu Arg Gly Asn Val Thr Val Gly Asp Phe Phe Ser Thr Ala Arg Ile
   145 150 155 160
   Gly Glu Asn Phe Gly Met Arg Gly Leu Arg Ile Ala Ser Asp Asp Arg
   165 170 175
   Met Leu Ala Pro Ser Gln Arg Gly Phe Ala Pro Val Val Arg Gly Val
   180 185 190
   Ala Asn Thr Asn Ala Lys Val Ser Ile Lys Gln Asn Gly Tyr Thr Ile
   195 200 205
   Tyr Gln Ile Thr Val Pro Ala Gly Pro Phe Val Ile Asn Asp Leu Tyr
   210 215 220
   Ala Ser Gly Tyr Ser Gly Asp Leu Thr Val Glu Ile Gln Glu Ser Asp
   225 230 235 240
   Gly Lys Val Arg Ser Phe Ile Val Pro Phe Ser Asn Leu Ala Pro Leu
   245 250 255
   Met Arg Val Gly His Leu Arg Tyr Gln Leu Ala Gly Gly Arg Tyr Arg
   260 265 270
   Ile Asp Ser Arg Thr Phe Asp Glu Arg Val Leu Gln Gly Val Leu Gln
   275 280 285
   Tyr Gly Leu Thr Asn His Leu Thr Leu Asn Ser Ser Leu Leu Tyr Thr
   290 295 300
   Arg His Tyr Arg Ala Gly Leu Phe Gly Phe Gly Leu Asn Thr Pro Ile
   305 310 315 320
   Gly Ala Phe Ser Ala Asp Ala Thr Trp Ser His Ala Glu Phe Pro Leu
   325 330 335
   Lys His Val Ser Lys Asn Gly Tyr Ser Leu His Gly Ser Tyr Ser Ile
   340 345 350
   Asn Phe Asn Glu Ser Gly Thr Asn Ile Thr Leu Ala Ala Tyr Arg Tyr
   355 360 365
   Ser Ser Arg Asp Phe Tyr Thr Leu Ser Asp Thr Ile Gly Leu Asn Arg
   370 375 380
   Thr Phe Arg Gln Phe Ser Gly Ala Tyr Leu Pro Glu Ile Tyr Arg Pro
   385 390 395 400
   Lys Asn Gln Phe Gln Val Ser Leu Ser Gln Ser Leu Gly Asn Trp Gly
   405 410 415
   Asn Leu Tyr Leu Ser Gly Gln Thr Tyr Asn Tyr Trp Glu Lys Arg Gly
   420 425 430
   Thr Asn Thr Gln Tyr Gln Val Ala Tyr Ser Asn Ser Phe His Ile Leu
   435 440 445
   Asn Tyr Ser Val Asn Leu Ser Gln Ser Ile Asp Lys Glu Thr Gly Lys
   450 455 460
   Arg Asp Asn Ser Ile Tyr Leu Ser Leu Ser Leu Pro Leu Gly Asp Asn
   465 470 475 480
   His Ser Ala Asp Ser Ser Tyr Ser Arg Ser Gly Asn Asp Ile Asn Gln
   485 490 495
   Arg Leu Gly Val Asn Gly Ser Phe Gly Glu Arg His Gln Trp Ser Tyr
   500 505 510
   Gly Ile Asn Ala Ser Arg Asn Asn Gln Gly Tyr Arg Ser Tyr Asp Gly
   515 520 525
   Asn Leu Ser His Asn Asn Ser Ile Gly Ser Tyr Arg Ala Ser Tyr Ser
   530 535 540
   Arg Asp Ser Leu Lys Asn Arg Ser Ile Ser Leu Gly Ala Ser Gly Ala
   545 550 555 560
   Val Val Ala His Lys His Gly Ile Thr Leu Ser Gln Pro Val Gly Glu
   565 570 575
   Ser Phe Ala Ile Ile His Ala Lys Asp Ala Ala Gly Ala Lys Val Glu
   580 585 590
   Ser Gly Ala Asn Val Ser Leu Asp Tyr Phe Gly Asn Ala Val Met Pro
   595 600 605
   Tyr Thr Ser Pro Tyr Glu Ile Asn Tyr Ile Gly Ile Asn Pro Ser Asp
   610 615 620
   Ala Glu Ala Asn Val Glu Phe Glu Ala Thr Glu Arg Gln Ile Ile Pro
   625 630 635 640
   Arg Ala Asn Ser Ile Ser Leu Val Asp Phe Arg Thr Gly Lys Asn Thr
   645 650 655
   Met Val Leu Phe Asn Leu Thr Leu Pro Asn Gly Glu Pro Val Pro Met
   660 665 670
   Ala Ser Thr Ala Gln Asp Ser Glu Gly Ala Phe Val Gly Asp Val Val
   675 680 685
   Gln Gly Gly Val Leu Phe Ala Asn Lys Leu Thr Gln Pro Lys Gly Glu
   690 695 700
   Leu Ile Val Lys Trp Gly Glu Arg Glu Ser Glu Gln Cys Arg Phe Gln
   705 710 715 720
   Tyr Gln Val Asp Leu Asp Asn Ala Gln Ile Gln Ser His Asp Ile Gln
   725 730 735
   Cys Lys Thr Ala Lys
   740
(2) INFORMATION FOR SEQ ID NO:8:
   (i) SEQUENCE CHARACTERISTICS:
      (A) LENGTH: 205 amino acids
      (B) TYPE: amino acid
      (D) TOPOLOGY: linear
   (ii) MOLECULE TYPE: protein
   (xi) SEQUENCE DESCRIPTION: SEQ ID NO:8:
   Met Gln Lys Thr Pro Lys Lys Leu Thr Ala Leu Phe His Gln Lys Ser
   1 5 10 15
   Thr Ala Thr Cys Ser Gly Ala Asn Tyr Ser Gly Ala Asn Tyr Ser Gly
   20 25 30
   Ser Lys Cys Phe Arg Phe His Arg Leu Ala Leu Leu Ala Cys Val Ala
   35 40 45
   Leu Leu Asp Cys Ile Val Ala Leu Pro Ala Tyr Ala Tyr Asp Gly Arg
   50 55 60
   Val Thr Phe Gln Gly Glu Ile Leu Ser Asp Gly Thr Cys Lys Ile Glu
   65 70 75 80
   Thr Asp Ser Gln Asn Arg Thr Val Thr Leu Pro Thr Val Gly Lys Ala
   85 90 95
   Asn Leu Ser His Ala Gly Gln Thr Ala Ala Pro Val Pro Phe Ser Ile
   100 105 110
   Thr Leu Lys Glu Cys Asn Ala Asp Asp Ala Met Lys Ala Asn Leu Leu
   115 120 125
   Phe Lys Gly Gly Asp Asn Thr Thr Gly Gln Ser Tyr Leu Ser Asn Lys
   130 135 140
   Ala Gly Asn Gly Lys Ala Thr Asn Val Gly Ile Gln Ile Val Lys Ala
   145 150 155 160
   Asp Gly Ile Gly Thr Pro Ile Lys Val Asp Gly Thr Glu Ala Asn Ser
   165 170 175
   Glu Lys Ala Pro Asp Thr Gly Lys Ala Gln Asn Gly Thr Val Ile Gln
   180 185 190
   Pro Arg Phe Gly Tyr Phe Gly Ser Leu Leu Arg His Arg
   195 200 205
(2) INFORMATION FOR SEQ ID NO:9:
   (i) SEQUENCE CHARACTERISTICS:
      (A) LENGTH: 437 amino acids
      (B) TYPE: amino acid
      (D) TOPOLOGY: linear
   (ii) MOLECULE TYPE: protein
   (xi) SEQUENCE DESCRIPTION: SEQ ID NO:9:
   Met Asn Lys Lys Ser Tyr Ile Asn His Tyr Leu Thr Leu Phe Lys Val
   1               5                   10                  15
   Thr Thr Leu Leu Phe Thr Leu Ser Ser Asn Pro Val Trp Ala Asn Ile
               20                  25                  30
   Lys Thr Val Gln Gly Thr Thr Ser Gly Phe Pro Leu Leu Thr Arg Thr
           35                  40                  45
   Phe Thr Phe Asn Gly Asn Leu Gln Trp Asn Val Ser Ala Leu Gln Pro
       50                  55                  60
   Ala Tyr Ile Val Ser Ser Gln Ala Arg Asp Asn Leu Asp Thr Val His
   65                  70                  75                  80
   Ile Gln Ser Ser Glu Ile Asn Ala Pro Thr Asn Ser Leu Ala Pro Phe
                   85                  90                  95
   Asn Asn Trp Ile Asn Thr Lys Ser Ala Val Glu Leu Gly Tyr Ser Phe
               100                 105                 110
   Ala Gly Ile Thr Cys Thr Ser Asn Pro Cys Pro Thr Met Lys Leu Pro
           115                 120                 125
   Leu Leu Phe His Pro Asp Leu Thr Asn Leu Thr Pro Pro Gly Lys Lys
       130                 135                 140
   Asn Ser Asp Gly Gly Glu Ile Phe Lys Leu His Asn Glu Ser Asn Leu
   145                 150                 155                 160
   Gly Val Ser Phe Gln Ile Gly Val Lys Thr Asn Thr Ser Leu Asp Trp
                   165                 170                 175
   Val Asn Ala Lys Asn Asn Phe Ser Ser Leu Lys Val Leu Met Val Pro
               180                 185                 190
   Phe Asn Ser Ser Asp Lys Ile Ser Leu His Leu Arg Ala Lys Phe His
           195                 200                 205
   Leu Leu Thr Asp Phe Ser Ser Leu Asn Asn Asp Ile Thr Ile Asp Pro
   210 215 220
   Met Asn Thr Ser Ile Gly Lys Ile Asn Leu Glu Thr Trp Arg Gly Ser
   225 230 235 240
   Thr Gly Asn Phe Ser Val Lys Tyr Val Gly Glu Asp Lys Gly Asp Ile
   245 250 255
   Ser Ile Phe Phe Asn Thr Pro Lys Ile Ile Leu Lys Lys Gln Gln Arg
   260 265 270
   Arg Cys Thr Leu Asn Asn Ala Pro Val Ser Pro Asn Pro Val Lys Leu
   275 280 285
   Arg Ala Val Lys Lys Arg Glu Leu Glu Ala Gln Ser Glu Met Glu Gly
   290 295 300
   Gly Thr Phe Gln Leu Arg Val Asn Cys Asp Asn Thr Thr Tyr Asn Lys
   305 310 315 320
   Ala Asn Gly Lys Trp Leu Phe Pro Val Val Lys Val Thr Phe Thr Asp
   325 330 335
   Glu Asp Gly Thr Thr Asn Asn Gly Thr Asn Asp Leu Leu Arg Thr Gln
   340 345 350
   Thr Gly Ser Gly Gln Ala Thr Gly Val Ser Leu Arg Ile Lys Arg Glu
   355 360 365
   Asn Gly Thr Glu Thr Val Lys Tyr Gly Ala Asp Ser Ala Gln Met Gly
   370 375 380
   Asn Ala Gly Gln Phe Glu Leu Arg Lys Gln Pro Ser Pro Ala Gly Gly
   385 390 395 400
   Asp Gln Tyr Ala Glu Glu Thr Phe Lys Val Tyr Tyr Val Lys Asp Ser
   405 410 415
   Thr Arg Gly Thr Leu Ile Glu Gly Lys Val Lys Ala Ala Ala Thr Phe
   420 425 430
   Thr Met Ser Tyr Gln
   435
(2) INFORMATION FOR SEQ ID NO:10:
   (i) SEQUENCE CHARACTERISTICS:
      (A) LENGTH: 316 amino acids
      (B) TYPE: amino acid
      (D) TOPOLOGY: linear
   (ii) MOLECULE TYPE: protein
   (xi) SEQUENCE DESCRIPTION: SEQ ID NO:10:
   Met Leu Ala Lys Ala Lys Tyr Arg Lys Asp Tyr Lys Gln Pro Asp Phe
    1               5                  10                  15
   Thr Val Thr Asp Ile Tyr Leu Asp Phe Gln Leu Asp Pro Lys Asn Thr
               20                  25                  30
   Val Val Thr Ala Thr Thr Lys Phe Gln Arg Leu Asn Asn Glu Ala Thr
           35                  40                  45
   Ser Leu Arg Leu Asp Gly His Ser Phe Gln Phe Ser Ser Ile Lys Phe
       50                  55                  60
   Asn Gly Glu Pro Phe Ser Asp Tyr Gln Gln Asp Gly Glu Ser Leu Thr
   65                  70                  75                  80
   Leu Asp Leu Lys Asp Lys Ser Ala Asp Glu Phe Glu Leu Glu Ile Val
                   85                  90                  95
   Thr Phe Leu Val Pro Ala Glu Asn Thr Ser Leu Gln Gly Leu Tyr Gln
               100                 105                 110
   Ser Gly Glu Gly Ile Cys Thr Gln Cys Glu Ala Glu Gly Phe Arg Gln
           115                 120                 125
   Ile Thr Tyr Met Leu Asp Arg Pro Asp Val Leu Ala Arg Tyr Ile Ile
       130                 135                 140
   Lys Ile Thr Ala Asp Lys Thr Lys Tyr Pro Phe Leu Leu Ser Asn Gly
   145 150 155 160
   Asn Arg Ile Ala Ser Gly Glu Leu Glu Asp Gly Arg His Trp Val Glu
   165 170 175
   Trp Asn Asp Pro Phe Pro Lys Pro Ser Tyr Leu Phe Ala Leu Val Ala
   180 185 190
   Gly Asp Xaa Gly Leu Leu Gln Asp Xaa Phe Ile Thr Lys Ser Gly Arg
   195 200 205
   Glu Val Ala Leu Glu Leu Tyr Val Asp Arg Gly Asn Leu Asn Arg Ala
   210 215 220
   Thr Gly Ala Met Glu Ser Leu Lys Lys Ala Met Lys Trp Asp Glu Asp
   225 230 235 240
   Arg Phe Ile Leu Glu Phe Tyr Leu Asp Ile Tyr Met Ile Ala Ala Ala
   245 250 255
   Asp Ser Ser Asn Met Gly Ala Met Glu Asn Lys Gly Leu Asn Ile Phe
   260 265 270
   Asn Ser Lys Leu Val Leu Ala Asn Pro Gln Thr Ala Thr Asp Glu Asp
   275 280 285
   Tyr Leu Val Ile Glu Ser Val Ile Ala His Glu Tyr Ser His Asn Trp
   290 295 300
   Thr Gly Asn Arg Val Thr Arg Arg Asp Gly Phe Asn
   305 310 315
(2) INFORMATION FOR SEQ ID NO:11:
   (i) SEQUENCE CHARACTERISTICS:
      (A) LENGTH: 670 amino acids
      (B) TYPE: amino acid
      (C) STRANDEDNESS: single
      (D) TOPOLOGY: linear
   (ii) MOLECULE TYPE: protein
   (xi) SEQUENCE DESCRIPTION: SEQ ID NO:11:
   Lys Ile Glu Glu Gly Lys Leu Val Ile Trp Ile Asn Gly Asp Lys Gly
   1 5 10 15
   Tyr Asn Gly Leu Ala Glu Val Gly Lys Lys Phe Glu Lys Asp Thr Gly
   20 25 30
   Ile Lys Val Thr Val Glu His Pro Asp Lys Leu Glu Glu Lys Phe Pro
   35 40 45
   Gln Val Ala Ala Thr Gly Asp Gly Pro Asp Ile Ile Phe Trp Ala His
   50 55 60
   Asp Arg Phe Gly Gly Tyr Ala Gln Ser Gly Leu Leu Ala Glu Ile Thr
   65 70 75 80
   Pro Asp Lys Ala Phe Gln Asp Lys Leu Tyr Pro Phe Thr Trp Asp Ala
   85 90 95
   Val Arg Tyr Asn Gly Lys Leu Ile Ala Tyr Pro Ile Ala Val Glu Ala
   100 105 110
   Leu Ser Leu Ile Tyr Asn Lys Asp Leu Leu Pro Asn Pro Pro Lys Thr
   115 120 125
   Trp Glu Glu Ile Pro Ala Leu Asp Lys Glu Leu Lys Ala Lys Gly Lys
   130 135 140
   Ser Ala Leu Met Phe Asn Leu Gln Glu Pro Tyr Phe Thr Trp Pro Leu
   145 150 155 160
   Ile Ala Ala Asp Gly Gly Tyr Ala Phe Lys Tyr Glu Asn Gly Lys Tyr
   165 170 175
   Asp Lys Ile Lys Asp Val Gly Val Asp Asn Ala Gly Ala Lys Ala Gly
   180 185 190
   Leu Thr Phe Leu Val Asp Leu Ile Lys Asn Lys His Met Asn Ala Asp
   195 200 205
   Thr Asp Tyr Ser Ile Ala Glu Ala Ala Phe Asn Lys Gly Glu Thr Ala
   210 215 220
   Met Thr Ile Asn Gly Pro Trp Ala Trp Ser Asn Ile Asp Thr Ser Lys
   225 230 235 240
   Val Asn Tyr Gly Val Thr Val Leu Pro Thr Phe Lys Gly Gln Pro Ser
   245 250 255
   Lys Pro Phe Val Gly Val Leu Ser Ala Gly Ile Asn Ala Ala Ser Pro
   260 265 270
   Asn Lys Glu Leu Ala Lys Glu Phe Leu Glu Asn Tyr Leu Leu Thr Asp
   275 280 285
   Glu Gly Leu Glu Ala Val Asn Lys Asp Lys Pro Leu Gly Ala Val Ala
   290 295 300
   Leu Lys Ser Tyr Glu Glu Glu Leu Ala Lys Asp Pro Arg Ile Ala Ala
   305 310 315 320
   Thr Met Glu Asn Ala Gln Lys Gly Glu Ile Met Pro Asn Ile Pro Gln
   325 330 335
   Met Ser Ala Phe Trp Tyr Ala Val Arg Thr Ala Val Ile Asn Ala Ala
   340 345 350
   Ser Gly Arg Gln Thr Val Asp Glu Ala Leu Lys Asp Ala Gln Thr Arg
   355 360 365
   Ile Thr Lys Ile Glu Gly Arg Thr Leu Ser Ser Asn Pro Val Trp Ala
   370 375 380
   Asn Ile Lys Thr Val Gly Thr Thr Ser Gly Phe Pro Leu Leu Thr Arg
   385 390 395 400
   Thr Phe Thr Glu Asn Gly Asn Leu Trp Asn Val Ser Ala Leu Pro Ala
   405 410 415
   Tyr Ile Val Ser Ser Ala Arg Asp Asn Leu Asp Thr Val His Ile Gln
   420 425 430
   Ser Ser Glu Ile Asn Ala Pro Thr Asn Ser Leu Ala Pro Glu Asn Asn
   435 440 445
   Trp Ile Asn Thr Lys Ser Ala Val Glu Leu Gly Tyr Ser Phe Ala Gly
   450 455 460
   Ile Thr Cys Thr Ser Asn Pro Cys Pro Thr Met Lys Leu Pro Leu Leu
   465 470 475 480
   Phe His Pro Leu Thr Asn Leu Thr Pro Pro Gly Lys Lys Asn Ser Asp
   485 490 495
   Gly Gly Glu Ile Phe Lys Leu His Asn Glu Ser Asn Leu Gly Val Ser
   500 505 510
   Phe Gln Ile Gly Val Lys Thr Asn Thr Ser Leu Asp Trp Val Asn Ala
   515 520 525
   Lys Asn Asn Phe Ser Ser Leu Lys Val Leu Met Val Pro Phe Asn Ser
   530 535 540
   Ser Lys Ser Ile Ser Leu His Leu Arg Ala Lys Phe His Leu Leu Thr
   545 550 555 560
   Asp Phe Ser Ser Leu Asn Asn Asp Ile Thr Ile Asp Pro Met Asn Thr
   565 570 575
   Ser Ile Gly Lys Ile Asn Leu Glu Thr Trp Arg Gly Ser Thr Gly Asn
   580 585 590
   Phe Ser Val Lys Tyr Val Gly Glu Asp Lys Gly Asp Ile Ser Ile Phe
   595 600 605
   Phe Asn Thr Pro Lys Ile Ile Leu Lys Lys Gln Gln Arg Arg Cys Thr
   610 615 620
   Leu Asn Asn Ala Pro Val Ser Pro Asn Pro Val Lys Leu Arg Ala Val
   625 630 635 640
   Lys Lys Arg Glu Leu Glu Ala Gln Ser Glu Met Glu Gly Gly Thr Phe
   645 650 655
   Leu Arg Val Asn Cys Asp Asn Thr Thr Tyr Asn Lys Ala Asn
   660 665 670
(2) INFORMATION FOR SEQ ID NO:12:
   (i) SEQUENCE CHARACTERISTICS:
      (A) LENGTH: 33 base pairs
      (B) TYPE: nucleic acid
      (C) STRANDEDNESS: single
      (D) TOPOLOGY: linear
   (xi) SEQUENCE DESCRIPTION: SEQ ID NO:12:
   GTGCTGGATC CGTTTCTCTT GCATTACATT AGG 33
(2) INFORMATION FOR SEQ ID NO:13:
   (i) SEQUENCE CHARACTERISTICS:
      (A) LENGTH: 34 base pairs
      (B) TYPE: nucleic acid
      (C) STRANDEDNESS: single
      (D) TOPOLOGY: linear
   (xi) SEQUENCE DESCRIPTION: SEQ ID NO:13:
   TTAGGAATTC GGAAGCGTTT TTTACTTTTT TTGG 34
(2) INFORMATION FOR SEQ ID NO:14:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (xi) SEQUENCE DESCRIPTION: SEQ ID NO:14:
   AACGAATTCT GCTGTTTATT AAGGCTTTAG 30
(2) INFORMATION FOR SEQ ID NO:15:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (xi) SEQUENCE DESCRIPTION: SEQ ID NO:15:
   AGCTGGATCC TTGTAGGGTG GGCGTAAGCC 30
(2) INFORMATION FOR SEQ ID NO:16:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (xi) SEQUENCE DESCRIPTION: SEQ ID NO:16:
   AGCTGGATCC TTGTAGGGTG GGCGTAAGCC 30
(2) INFORMATION FOR SEQ ID NO:17:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (xi) SEQUENCE DESCRIPTION: SEQ ID NO:17:
   AACGGATTCG TTTGCTGTTT ATTAAGCCTT 30
(2) INFORMATION FOR SEQ ID NO:18:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (xi) SEQUENCE DESCRIPTION: SEQ ID NO:18:
   AACGGATTCG TTTGCTGTTT ATTAAGCCTT 30
(2) INFORMATION FOR SEQ ID NO:19:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (xi) SEQUENCE DESCRIPTION: SEQ ID NO:19:
   GGCAAATACG CACCGCTAAA T                                    21
(2) INFORMATION FOR SEQ ID NO:20:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (xi) SEQUENCE DESCRIPTION: SEQ ID NO:20:
   CGGACGAAGA TGGTACAACG A                                                 21
(2) INFORMATION FOR SEQ ID NO:21:
   (i) SEQUENCE CHARACTERISTICS:
      (A) LENGTH: 34 base pairs
      (B) TYPE: nucleic acid
      (C) STRANDEDNESS: single
      (D) TOPOLOGY: linear
   (xi) SEQUENCE DESCRIPTION: SEQ ID NO:21:
   CCAAGCTTGG CCCGACATTA TTATTGATAT GACA                                   34

## Description

Nontypable *Haemophilus influenzae* (NTHi) are primarily noninvasive human respiratory tract pathogens. NTHi can reside in the respiratory tract as a commensal or give rise to local infections, including otitis media, bronchitis, sinusitis, and rarely, pneumonia (Bluestone, C.D., and J.O. Klein, *In Pediatric Otolaryngology., 356* (1983); Bluestone and Stool ed. W.B. Saunders Co. Philadelphia.; Musher, D.M. *et al*., *Ann. Intern. Med. 99*:344-350 (1983)). Several potential adherence factors have been described for *Haemophilus influenzae* (both typable and nontypable) adherence to human cells, including four classes of fimbriae/pili and two high molecular weight proteins with similarity to the filamentous hemagglutinin of *Bordetella pertussis* (St. Geme, J.W., *et al*., *Proc. Natl. Acad. Sci. USA 90*:2875-2879 (1993)). Pili are bacterial surface antigens. They are protein appendages consisting of a helically symmetrical assembly of major protein (pilin) subunits. Some pili can also carry from two to three minor proteins assembled on their tips. One of these proteins, adhesin, carries the active site for pilus adhesion to specific membrane receptors on human and animal cells.

One class of pili/fimbriae has been widely studied, the long thick pili (LKP) family. LKP pili are expressed by both typable and nontypable *H. influenzae* (Hib). The pili in this family have a characteristic morphology, partially shared adhesion specificity and their structural proteins share amino acid sequences. These pili are hemagglutination positive and mediate attachment to human mucosal cells (Brinton, C.C. *et al., Pediatr. Infect. Dis. J. 8 Suppl*.:54-61 (1989)). Hemagglutination of human erythrocytes is accomplished via binding to the AnWj blood group antigen while binding to epithelial cells involves a sialic acid containing lactosylceramide receptor (van Alphen, L. *et al., Infect. Immun. 69*:4473-4477 (1991)).

The LKP family has been divided into different strain specific serotypes based on reactivity to polyclonal antisera raised against the purified pili. Little cross reactivity among pili serotypes has been observed (Brinton, C.C., *et al*., *Pediatr. Infect. Dis. J. 8 Suppl*.:54-61 (1989)).

Inhibiting, or blocking, LKP pilus-mediated adhesion by *H. influenzae* to cells can prevent *H. influenzae* diseases. Purified, intact LKP pili have been shown to be vaccine candidates for NTHi otitis media in the chinchilla model, conferring protection against challenge with NTHi strains bearing homologous pili serotype (Karasic, R. *et al*., *Pediatr. Infect. Dis. J. 8 (Suppl.)*: S62-65 (1988)). However, because protection is pilus-specific, for broad protection, a vaccine would be required to be multivalent, including the most frequently occurring serotypes of pili in the natural population of pathogens. LKP pilin structural genes have been cloned and sequenced by several groups (Coleman, T. *et al., Infect. Immun. 59*:1716-1722 (1991); Forney, L.J. *et al., Infect. Immun. 59*:1991-1996 (1991); Kar, S., *et al.Infect. Immun. 58*:903-908 (1990); van Ham, S.M., *et al., EMBO Jour. 8*:3535-3540 (1989)), but only the genes responsible for pili serotypes 1 and 4 have been identified.

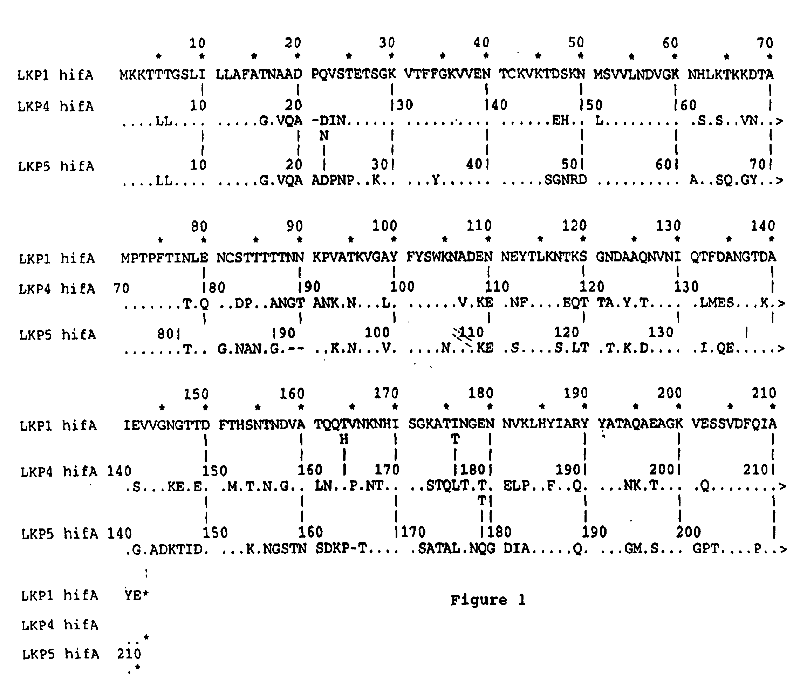

We describe the isolation, cloning and sequencing of the pilin gene for the *Haemophilus influenzae* pili serotype 5 (Figure 1), the sequencing of the entire LKP1 operon, which is set forth in Table 5 and the cloning of the LKP10, LKP11, and LKP12 pili. We describe DNA molecules (also referred to herein as DNA sequences or nucleic acid sequences) which encode proteins which comprise the *H. influenzae* LKP, particularly a tip adhesin protein. We also describe DNA molecules capable of hybridizing to the DNA sequences of the *Haemophilus influenzae* genome related to the pili. The DNA molecules described here can be used in a method for assaying a sample, such as a blood sample, for the presence of *Haemophilus influenzae*. The DNA molecules described here can accordingly be used as a diagnostic.

Recombinant *Haemophilus influenzae* pili proteins, and peptides, specifically a tip adhesin protein are also described The proteins or peptides can be used to produce antibodies, both polyclonal and monoclonal, which are reactive with (i.e., bind to) the *H*. *influenzae* pili proteins, and can be used in diagnostic assays to detect the presence of *Haemophilus influenzae* antibodies, in for example, a blood sample. Such antibodies can also be used as vaccines in methods of passive immunization.

The proteins and peptides described can also be employed in methods for immunizing a mammal, such as a human, against *Haemophilus influenzae* infection and, thus, as a vaccine for the prevention of *Haemophilus influenzae* related diseases, for example, otitis media. In particular, based on the DNA and amino acid sequences presented herein, an adhesin protein, or peptide, vaccine can be constructed which can induce protecting antibodies to *H. influenzae* in mammals.
Figure 1 is a graphic illustration of the conserved regions of the proteins encoded by pilin genes of *H. influenzae* serotypes 1, 4 and 5 (SEQ ID NOs:1-3, respectively).
Figures 2A, 2B, and 2C are schematics of the physical maps obtained by restriction enzyme digestion of vectors containing LKP inserts.

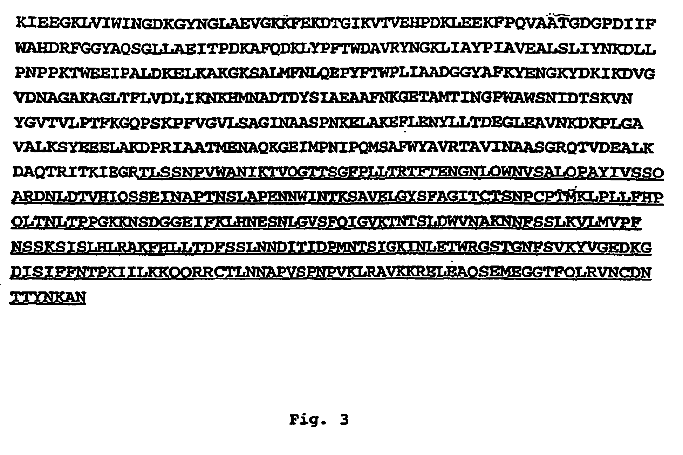

Figure 3 shows the amino acid sequence of LKP1 fusion protein. The underline indicates the partial amino acid sequence of the LKP tip adhesin protein that was fused to maltose-binding protein.
Figure 4 is a photograph of a gel showing the identification of LKP1 tip adhesin protein by antibodies reactive with the fusion protein of LKP1 tip adhesin-MBP in Western blotted membranes. Lanes 1 and 2: different preps of purified LKP1 pili with tip protein (47Kd). (A positive reaction was shown between tip protein and the antibody); lane 3: purified LKP10 pili with tip adhesin (47Kd). (The tip protein does not react with the antibody); lane 4: purified LKP11 pili with tip protein (47Kd). (The tip protein does not react with the antibody); lane 5: protein molecular weight markers.

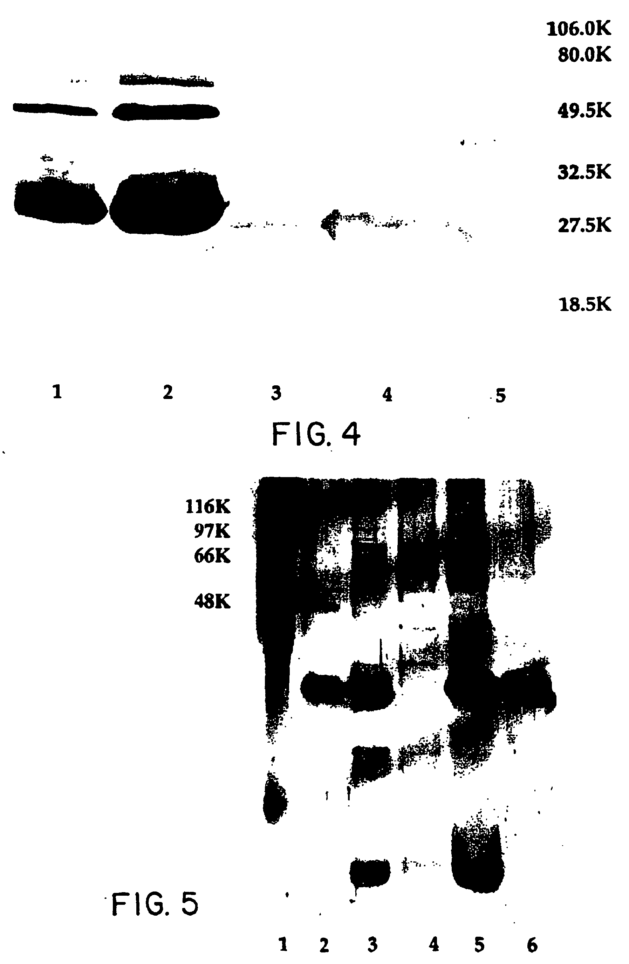

Figure 5 is a photograph of a gel showing the binding activity of LKP1 tip adhesin to human red cell (HRC) ghosts. Lane 1: molecular weight markers; lane 2: purified LKP1 pili with tip protein; lane 3: the pili with HRC ghosts after centrifugation. Tip protein band (47Kd) disappeared due to the binding of tip adhesin pili to ghosts pellet; lane 4: HRC ghosts after centrifugation, used as control; lane 5: purified pili without tip protein (treated with 1% SDS) was incubated with fresh ghosts, showing the same protein band pattern as the pattern of lane 3; Lane 6: purified pili without tip protein. Prior to the gel loading, pili were treated with 1% SDS, exhaustively dialyzed in 25 m Tris buffer, pH 8.0, crystallized by PEG plus NaCl and resolubilized in 25 mM Tris buffer, pH 8.0.

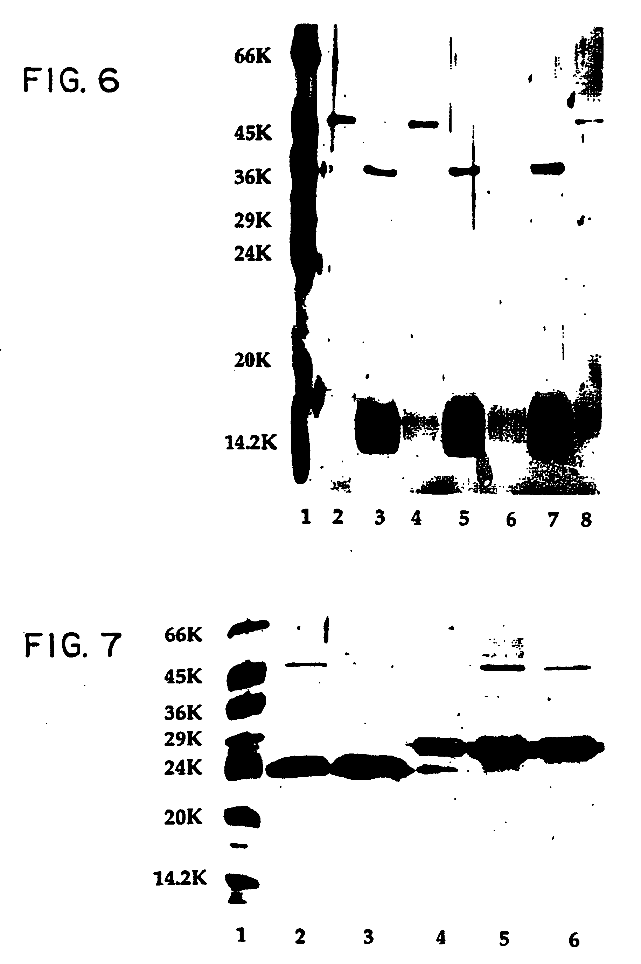

Figure 6 is a photograph of a gel showing the binding activity of purified LKP1 tip adhesin protein to human red cell ghosts. Lane 1: molecular weight markers; lane 2: purified tip adhesin protein with a molecular weight of 47Kd and the protein was removed by 0.1% SDS in 100 mM Glycine buffer, pH 2.0; lane 3: purified adhesin was incubated with fresh human red cell ghosts and pelleted by centrifugation prior to loading the supernatant on the gel. The tip adhesin band disappeared due to the binding to HRC ghosts; lane 4: purified adhesin was incubated with boiled HRC ghosts and pelleted by centrifugation prior to loading the supernatant on the gel. It showed adhesin band with 47Kd, which indicates that tip adhesin protein does not bind to the ghosts pellet; lane 5: supernatant of fresh ghosts after centrifugation. It was used as a control; lane 6: supernatant of boiled HRC ghosts after centrifugation, showing a different soluble protein pattern from that of fresh HRC ghosts, used as another control; lane 7: different prep of purified tip protein incubated with fresh HRC ghosts, which showed the binding between tip protein and fresh HRC ghosts pellet; lane 8: different prep of purified tip protein incubated with boiled HRC ghosts, indicating that the tip protein does not bind the denatured ghosts. The gel was silver stained.
Figure 7 is a photograph of a gel showing adhesin proteins from different LKP type pili with the same molecular weight. Lane 1: molecular weight markers; lane 2: LKP10 pili; lane 3: LKP11 pili and lane 4 to 6: different purified preparation of LKP1 pili. Proteins were stained with silver.

Described herein, for the first time, is the cloning of the *Haemophilus influenzae* serotype 5 pilin gene and the sequence of the entire LKP1 operon (Table 5/SEQ ID NO: 4) and the deduced amino acid sequences for six open reading frames (SEQ ID NOs: 5-10). The LKP1 operon, as shown in Table 5, is composed of five separate genes, designated *hipP* (the pilin gene), *hipC* (the periplasmic chaperone gene), *hipR* (the membrane anchor gene), *hipM* (the minor tip associated protein gene) and *hipA* (the tip adhesin gene). These five genes are also referred to herein as *hifA* (for *hipP*), *hifB* (for *hipC*), *hifC* (for *hipR*), *hifD* (for *hipM*) and *hifE* (for *hipA*). Also present on the LXP1 operon are an integrase gene, and a peptidase gene. The proteins encoded by these genes of the LKP1 operon and the LKP5 pilin protein are collectively referred to herein as the *H. influenzae* pili proteins.

Isolated and/or recombinant nucleic acid sequences encoding the *H. influenzae* pili proteins, or biologically active fragments thereof, are described herein. As used herein nucleic acids are also referred to as DNA and RNA, or DNA sequences and RNA sequences, or DNA molecules or RNA molecules. Nucleic acids referred to herein as "isolated" are nucleic acids separated away from the nucleic acids of the genomic DNA or cellular RNA of their source of origin (e.g., as it exists in cells or in a mixture of nucleic acids such as a library), and may have undergone further processing. "Isolated" nucleic acids include nucleic acids obtained by methods known to those of skill in the art to obtain isolated nucleic acids and methods described herein. These isolated nucleic acids include essentially pure nucleic acids, nucleic acids produced by chemical synthesis, by combinations of biological and chemical methods, and recombinant nucleic acids which are isolated.

Nucleic acids referred to herein as "recombinant" are nucleic acids which have been produced by recombinant DNA methodology, including those nucleic acids that are generated by procedures which rely upon a method of artificial recombination, such as the polymerase chain reaction (PCR) and/or cloning into a vector using restriction enzymes. "Recombinant" nucleic acids are also those that result from recombination events that occur through the natural mechanisms of cells, but are selected for after the introduction to the cells of nucleic acids designed to allow and make probable a desired recombination event.

Also described here are nucleic acid sequences (DNA or RNA sequences) which are substantially complementary to the *H. influenzae* DNA sequences described herein, and nucleic acid sequences which hybridize with these DNA sequences under conditions of stringency known to those of skill in the art sufficient to identify DNA sequences with substantial nucleic acid sequence identity. It is reasonable to predict that DNA sequences identified under such stringent conditions will likely encode a protein (also referenced to herein as a polypeptide, or peptide fragment) with the biological activity of *H. influenzae* pili proteins. A general description of stringent hybridization conditions are discussed in Ausubel, F.M., *et al*., Current Protocols in Molecular Biology, Greene Publishing Assoc. and Wiley-Interscience 1989. Factors such as probe length, base composition, percent mismatch between the hybridizing sequences, temperature and ionic strength influence the stability of nucleic acid hybrids. Thus, stringency conditions sufficient to identify additional *H. influenzae* pili proteins, (e.g., high or moderate stringency conditions) can be determined empirically, depending in part upon the characteristics of the known DNA to which other unknown nucleic acids are being compared for sequence similarity.

As defined herein, substantially complementary means that the sequence need not reflect the exact sequence of e.g., Table 5 (SEQ ID NO: 4), but must be sufficiently similar in identity of sequence to hybridize with Table 5 (SEQ ID NO: 4) under stringent conditions. For example, non-complementary bases, or longer or shorter sequences can be interspersed in sequences provided the sequence has sufficient complementary bases with, e.g., Table 5 (SEQ ID NO: 4) to hybridize therewith.

The DNA molecules can, preferably, encode a functional or biologically active pili protein, such as the pilin gene, *hipP;* the periplasmic chaperon, *hipC*; the membrane anchor protein, *hipR*; the tip associated protein, *hipM* and as described herein, the tip adhesin protein, *hipP*. A " functional or biologically active protein" is defined herein as a protein which shares significant identity (e.g., at least about 65%, preferably at least about 80% and most preferably at least about 95%) with the corresponding sequences of the endogenous protein and possesses one or more of the functions thereof. Biological functions of the *H. influenzae* pili proteins include antigenic structural, and adhesion properties. For example, as described in Karasic, R. *et al*. (Karasic, *R. et al*., *Pediatr. Infect. Dis. J. 8 (Suppl.)*: S62-65 (1988)), pili proteins can be shown to adhere to mucosal cells and erythrocytes. Thus, such adhesion properties,such as binding the red blood cells, can be a measure of biological activity. Also described herein, biological activity can include the antigenicity of the protein, or peptide, resulting in the production of antibodies which can bind to the pili proteins.

The *H. influenzae* pili proteins include the proteins of the LKP1 operon and the serotype 5 *hipP* pilin protein, and proteins having amino acid sequences analogous to these sequences. Such proteins are defined herein as *H. influenzae* pili protein analogs, or derivatives. Analogous amino acid sequences are defined herein to mean amino acid sequences with sufficient identity of amino acid sequence with, e.g., LKP1 tip adhesin protein, to possess the biological activity of tip adhesin. The biological activity of tip adhesin can include, for example, the capability of tip adhesin to bind to specific membrane receptors on human and animal cells, including binding to native red blood cell ghost preparations.

For example, an analog polypeptide can be produced with "silent" changes in the amino acid sequence wherein one, or more amino acid residue differs from the amino acid residues of the LKP1 adhesin, yet still possess adhesion activity. Examples of such differences include additions, deletions or substitutions of residues.

Also encompassed by the present invention are analogous proteins that exhibit lesser or greater biological activity of the pili proteins of the present invention.

We also describe biologically active protein, or biologically active fragments of the *H. influenzae* pili proteins. Such fragments can include only a part of the full-length amino acid sequence of a pili protein yet possess biological activity. Such fragments can be produced by amino- and carboxyl-terminal deletions, as well as internal deletions. Such peptide fragments can be tested for biological activity as described herein. Thus, a functional, or biologically active, protein includes mutants or derivatives of the endogenous protein wherein one or more amino acids have been substituted, deleted or added. Also described are active fragments of the protein. The *H. influenzae* pili proteins, include functional or biologically active pili proteins, such as the pilin structural protein, *hipP*; the periplasmic chaperon, *hipC*; the membrane anchor protein, *hipR*; the tip associated protein, *hipM*; and most preferably, as described herein, the tip adhesin protein, *hipA.* Fusion proteins comprising the pili proteins described herein (referred to herein as a first moiety) are linked to a second moiety not occurring in the pili protein as found in nature. Thus, the second moiety can be a single amino acid, peptide or polypeptide. The first moiety can be in an N-terminal location, a C-terminal location or internal to the fusion protein.

The DNA sequences described here can also be used in a recombinant construct for the infection, transfection or transformation of a cell *in vitro* or *in vivo* under control of an appropriate promoter for the expression of functional *H. influenzae pili* proteins, as defined herein, in an appropriate host cell. Such recombinant constructs are also referred to herein as expression vectors. For example, a DNA sequence can be functionally ligated to a suitable promoter (e.g., a constitutive or inducible promoter or the endogenous promoter) introduced into a suitable expression vector, such as pUC19, which is then introduced into a suitable host cell. The construct can also include DNA encoding one or more selectable markers (such as neo, gpt, dhfr, ada, pac, hyg and hisd) or DNA encoding one or more different antigens or therapeutic proteins.

The construct can be introduced by any suitable means, as set forth above, such as by calcium phosphate precipitation, microinjection, electroporation or infection (such as with an infectious retroviral, herpes vaccinia or adenovirus vector). The host cell can be a eucaryotic or procaryotic cell. Suitable cells include bacterial (e.g. *E. coli*) or mammalian cells. Mammalian cells include primary somatic cells, such as, epithelial cells, fibroblasts, keratinocytes, macrophages or T cells, or immortalized cell lines, such as HeLa or HT1080. The recombinant host cell can then be cultured and, optionally, selected, *in vitro* under appropriate conditions resulting in the expression of the protein. Alternatively, the cell can be transplanted or injected into an animal, such as a human, for in vivo expression.

One embodiment relates to LKP type pili-producing *E. coli* recombinants. Such recombinants have been constructed from *Haemophilus infuenzae*, as described herein. These single serotype recombinants produced pili in large, easily purifiable quantities. They did not phase vary or become recalcitrant upon subculture and could be grown as *E. coli* in liquid medium with good pilus yields. The single serotype pilus preparations grown and purified from them contained pili identical to those on the parent *H. influenzae (Hflu)* strains and contained no other *Hflu* antigens. These preparations are easily standardized for purity, identity, concentration and potency for subsequent mixing into a multivalent vaccine and provides an efficient means of producing pilus for vaccine manufacture. As described herein, single-type-producing *E. coli* recombinant vaccine strains have been constructed for LKP10, LKP11 and LKP12 serotypes.

Multiple serotype recombinants containing two operons on separate plasmids have also been constructed. Single colonies of these strains simultaneously expressed, in good quantities, two serotypes of pili. However, these strains were unstable in that, during in vitro subculture, they tended to rapidly lose pilus expression, perhaps because the plasmids used were incompatible. When the two operons are placed on two compatible plasmids these strains are expected to be more stable. The use of stable, high-producing double-expressing recombinant strains could simplify production of proteins suitable for vaccine use by reducing by half the number of vaccine strains required.

Good production, concentration and purification methods for *Hflu* LKP pili of different serotypes have been developed and are described herein. Pili can be purified from *E. coli* recombinant cultures producing *Hflu* pili as described for the purification of pili from *Hflu* culture. Both solid phase and liquid phase fermentation methods have been used. The preferred procedure involves mechanical removal of pili from the harvested bacteria and their separation from the bacterial cells by centrifugation Pili are concentrated and further purified by alternate cycles of longitudinal aggregation (crystallization) of intact pilus rods with soluble impurities removed by centrifugation of the crystals followed by solubilization of the pilus crystals into free pilus rods with particulate impurities removed by centrifugation. Each stage of the production/purification process was optimized for each pilus serotype. To date, nineteen different LKP serotypes have been purified.

Alternative pilus purification methods with analytical and industrial utility have also been developed. Using appropriate solvent and column conditions, intact pili can be purified away from contaminating proteins by HPLC or FPLC on molecular sizing, hydrophobic or ion exchange columns. These methods are also capable of scale-up for industrial production.

Purification methods for individual pilus proteins have also been developed starting with intact LKP pili. *Hflu* LKP pilus structural proteins, as deduced from the multiple sequence alignment of pilus gene sequences with other pilus genes, include pilin, small tip minor and large tip minor proteins. The large tip minor protein is referred to as the "adhesin" because it carries the known LKP pilus adhesion specificity for human red blood cells. However, by analogy with other pilus families, the other two LKP pilus structural proteins may also be adhesins with specificities for as yet unknown human receptors. Both pilins and adhesins of LKP pili have been purified in biologically active form.

The pilins are purified in assembled rod form by removal of the minor tip proteins and separation of rods from minors on molecular sizing columns. In their assembled form, the pilin units retain the antigenic specificity of intact pili which is conferred by the exposed surface determinants of the pilin subunits on the lateral surface of the pilus rod. Pilin rods are expected to be equally as effective multivalent vaccine components as intact pili may have advantage of higher purity and possibly reduced side effects.

The adhesin of LKP11 has been isolated and purified active and soluble form. Its removal from LKP11 pili eliminates the ability of these pili to bind to human red blood cells. In pure form it can bind to human red blood cell membranes. The adhesin band on SDS gels is labeled by antibodies reactive with fusion protein comprised a fragment of adhesin and maltose binding protein. Purified LKP pilus adhesins may have utility as vaccine components capable of inducing adhesion-blocking or clearing antibodies. The LKP11 adhesin did not cross-react antigenically with the LKP1 adhesin on Western blots. Thus, the SDS/PAGE gel similarity of apparent molecular weights found for 3 different LKP adhesins was not predictive of antigenic similarity in this limited two-serotype test. Free adhesins can be tested for efficacy as otitis media vaccines and for their ability to induce adhesion-blocking antibodies. Antiserum to the fusion protein, which labeled the adhesin band on Western blots, did not block adhesion to red cells.

The isolated recombinant proteins of the present invention can be administered to a mammal to protect, or to treat the mammal against *H. influenzae* infection. Isolated recombinant pili protein can be formulated into a vaccine composition, for example, as described in U.S. Patent 5,336,490. The protein can also be administered via an infectious construct, preferably a replication incompetent or attenuated viral construct. Alternatively, the protein can be administered via a recombinant host cell (such as, a mammalian cell) which will express the protein *in vivo* or in a pharmaceutically acceptable carrier. In particular, the recombinant LKP1 tip adhesin protein, a biologically active fragment thereof, or a fusion protein, can be used in a vaccine composition to induce the production of antibodies in a mammal. It is reasonable to predict that such antibodies can protect the mammal from *H. influenzae* diseases.

The vaccine composition may be administered in a single dose or in more than one dose over a period of time to achieve a level of antibody in the blood which is sufficient to confer protection from *H. influenzae* infection.

Suitable pharmaceutical carriers include, but are not limited to water, salt solutions, alcohols, polyethylene glycols, gelatin, carbohydrates such as lactose, amylose or starch, magnesium stearate, talc, silicic acid, viscous paraffin, fatty acid esters, hydroxymethylcellulose, polyvinyl pyrolidone, etc. The pharmaceutical preparations can be sterilized and if desired, mixed with auxiliary agents, e.g., lubricants, preservatives, stabilizers, wetting agents, emulsifiers, salts for influencing osmotic pressure, buffers, coloring, and/or aromatic substances and the like which do not deleteriously react with the active compounds. They can also be combined where desired with other active agents, e.g., enzyme inhibitors, to reduce metabolic degradation.

For parenteral application, particularly suitable are injectable, sterile solutions, preferably oily or aqueous solutions, as well as suspensions, emulsions, or implants, including suppositories. Ampoules are convenient unit dosages.

Modes of administration are those known in the art, such as parenteral, oral or intranasal administration or by cellular implantation.

It will be appreciated that the actual effective amounts of the protein in a specific case will vary according to the specific compound being utilized, the particular composition formulated, the mode of administration and the age, weight and condition of the patient, for example. As used herein, an effective amount of protein is an amount of protein which is capable of raising the level of antibody in a mammal to a level sufficient to provide protection from *H. influenzae* infection. Dosages for a particular patient can be determined by one of ordinary skill in the art using conventional considerations, (e.g. by means of an appropriate, conventional pharmacological protocol).

The DNA molecules and proteins described here can be used in *in vitro* diagnostic assays to detect the presence of *H. influenzae* in biological samples. These DNA molecules, or fragments thereof, can be used as probes in an assay for detecting *Haemophilus influenzae* in a sample, such as a blood sample from a mammal, e.g. a human. Such probes can be designed such that they specifically bind to the target sequence (e.g., an *H. influenzae* pili protein).

In one embodiment the DNA probe can comprise the nucleotides of a serotype conserved region of the *H. influenzae* genome, such as the nucleotides encoding a tip adhesin protein. To specifically bind to the target sequence, the probe must be of sufficient length to provide the desired specificity i.e., to avoid being hybridized to random sequences in the sample. The DNA molecule capable of hybridization preferably contains at least about 400 nucleotides, more preferably at least about 1000 nucleotides, and most preferably at least about 1200 nucleotides. For example, the DNA molecule can comprise at least about 400 nucleotides between about nucleotide 7000 to 7400 of Table 5 (SEQ ID NO: 4). The DNA hybridization probe preferably shares at least about around 70% homology or the corresponding sequences of the *Haemophilus influenzae* genome, more preferably at least about 80% and most preferably at least about 90%.

In particular, the DNA molecules descri bed here are capable of hybridizing to serotype conserved regions of the *H. influenzae* genome. A particularly preferred embodiment are DNA molecules that hybridize with the *H. influenzae* region encoding the tip adhesin protein. For example, a DNA molecule can be capable of hybridizing to the gene encoding the tip adhesin protein of serotype 1, preferably the sequence set forth between about nucleotide 6955 to 8265 of Table 5 (SEQ ID NO: 4). In one embodiment, the DNA molecule is capable of hybridizing to the genome under stringent conditions, as described herein. The hybridization assay can be performed employing known hybridization procedures, such as those described herein. The probe can be, for example, detectably labeled employing known labels in the art, including enzymes, dyes, antibodies and radioactive labels. The probe is preferably immobilized on a solid support (e.g., a membrane).

Alternatively, the DNA molecule can be selected such that it hybridizes to a non-conserved region of the *Haemophilus influenzae* genome. For example, a DNA molecule that hybridizes to the gene encoding the pilin protein can be employed. Such an assay can detect the presence of a particular serotype of *Haemophilus influenzae* in the sample.

A sample which can be subjected to the present assay can be any sample which is suspected of containing or being contaminated with *Haemophilus influenzae.* Examples of such an sample include a blood sample, a nasopharyngeal sample, or an ear aspirate.

The assay can be used, therefore, as a diagnostic for the detection of infection of a subject, such as a mammal (e.g., a human), with *Haemophilus influenzae*. The assay can also be used to detect the presence of contamination of a material with *Haemophilus influenzae*, such as a food, medicament, or biological material.

The protein can be used in an assay for detecting *Haemophilus influenzae* infection in a sample, such as a blood sample. For example, the pili of a pathogen can be isolated from the sample or recombinantly produced, employing the techniques described herein. One or more of the proteins, or fragments thereof, of the pili can then be sequenced. The sequences can be aligned to and compared with the corresponding protein sequence(s) of SEQ ID NO:4. Homology in excess of 90%, for example, is indicative of presence of the pathogen (i.e., infection) in the sample.

The pili protein, or a fragment thereof (e.g., a peptide fragment) can also be used in an immunoassay, specifically an ELISA, to detect the presence of antibodies in biological samples (e.g., blood, serum or tissue). Such immunoassay can be readily performed by those of skill in the art using well-established techniques to detect antibody bound to LKP pili protein or peptide fragments.

The pili proteins, or fragments thereof (also referred to herein as peptides, or peptide fragments), can also be used to produce antibodies that are reactive with the pili proteins described herein. The term antibody is intended to encompass both polyclonal and monoclonal antibodies. Polyclonal antibodies can be prepared by immunizing an animal with a preparation of crude or purified pili protein using techniques well-known to those of skill in the art. Pili fusion proteins can also be used for immunization. Monoclonal antibodies can be prepared using techniques known to those of skill in the art. These antibodies can be used in diagnostic assays to detect the presence of *H. influenzae* antibodies in biological samples as described above.

The invention is further specifically illustrated by the following examples.

### Example 1: Cloning and Sequencing of the LKP 5, hipP gene and the LKP1 Operon

### Materials and Methods

### Bacterial strains and plasmids

*H. influenzae* strains P860295, P861249, and P860688 which express LKP serotypes 1, 4, and 5 respectively, described previously (Brinton, C.C. *et al., Pediatr. Infect. Dis. J. 8 Suppl.*: 54-61 (1989)) were employed. *E. coli* strains MB392 (Kar, S. *et al., Infect. Immun. 58*:903-908 (1990)) and HB101 were used as hosts for recombinant plasmids and strain DH5-α was used for cloning steps involving β-galactosidase α-peptide complementation. *Hflu* were grown in brain heart infusion (Dco Laboratories, Detroit, MI) containing 10 µg/ml hemin (Sigma Chemical Co., St. Louis, MO) and 2 µg/ml NAD (Sigma) at 37° C. *E. coli* strains were grown in Luria broth (Miller, J.H., In *Experiments in molecular genetics.,* 203 (1972). Cold Spring Harbor Laboratory. Cold Spring Harbor, NY) at 37°C. Where appropriate, antibiotics were used at the following concentrations: ampicillin (Sigma) 100 pg/ml, kanamycin (Sigma) 25 µg/ml, and chloramphenicol (Sigma) 20 µg/ml.

Construction and properties of plasmid pHF₁ which expresses LKP1 pili in *E. coli* as described previously (Kar, S. *et al., Infect. Immun. 58*:903-908 (1990)) were employed. Plasmid pPX551 is a pUC18 derivative containing the 1.9 kb XhoI fragment of pHF1 inserted into the BamHI site. Deletion clones of pHF1 lacking the pepN locus were constructed as described in the text. The LKP4 pilin structural gene was isolated by PCR amplification of P860295 chromosomal DNA using primers with the following sequences: for the 5' end of the gene-5'GTGCTGGATCCGTTTCTCTTGCATTACATTAGG 3' (SEQ ID NO:12) and for the 3' end- 5'TTAGGAATTCGGAAGCGTTTTTTACTTTTTTTGG3' (SEQ ID NO:13). The 5' primer included a *Hin*dIII restriction site, underlined in the sequence, and the 3' primer included an *Eco*RI site also shown underlined. The PCR product was cloned into pCR1000 (Invitrogen, Inc., CA) as per manufacturer's directions. The LKP4 structural gene was subcloned by blunting the *Eco*RI site with Klenow in the presence of all four dNTPs, and cutting with *Asp*718 I (an *Asp*718 I site is located in the vector) releasing the fragment. The LKP4 gene was ligated into *Hin*dII-*Asp*718 I cut pPX191 (a derivative of pUC19 with the *bla* gene replaced by the cat gene from pACYC184 (Chang, A.C.Y., and S.N. Cohen, *J. Bacteriol. 134*:1141-1156 (1978)) to form pPX602.

The LKP5 pilin structural gene was isolated by PCR using the following primers: for the 5'end- 5'-AACGAATTCTGCTGTTTATTAAGGCTTTAG (SEQ ID NO:14) and for the 3'-AGCTGGATCCTTGTAGGGTGGGCGTAAGCC (SEQ ID NO:15). The PCR product of approximately 1 kb was cloned into pCRII (Invitrogen, Inc., San Diego, CA and subcloned as a blunt ended fragment by Klenow treatment of *Eco*RI ends generated using the vector's flanking *Eco*RI sites. The LKP5 pilin gene was subcloned into plasmid pPX191 and orientation determined by restriction analysis. The LKP5 subclone was saved as pPX605.

### Cloning of hipP genes encoding other LKP serotypes

*hipP* loci encoding serotype 4 and serotype 1 LKP genes have been described (Kar, S. *et al., Infect. Immun. 58*:903-908 (1990); van Ham, S.M. *et al., EMBO Jour. 8*:3535-3540 (1989)). To determine the serotype specificity of LKP pili is located within the *hipP* gene, PCR was used to clone the serotypes 4 and 5 pilin genes from an NTHi strains expressing these pili. The PCR product for the LKP4 pilin gene was cloned into pPX191 as described above and is expressed under control of the lac promoter. The *hipP* gene from an LKP5 expressing *Hflu* strain was isolated by PCR as described and cloned into pPX191 for expression under lac control.

### Oligonucleotide synthesis

The synthetic oligonucleotides used as primers for PCR amplification and DNA sequencing were synthesized on an Applied Biosystems (ABI) 380B DNA synthesizer using b-cyanoethyl phosphoramidite chemistry (Sinha, N.D. *et al., Nucleic Acids Research 12*:4539-4557 (1984)).

### Polymerase chain reaction (PCR) amplification

The LKP4 *hip*P and LKP5 *hipP* pilin genes were amplified by PCR from NTHi strains P861249 and P860688 respectively, using standard PCR amplification protocols (Saiki, R.K. *et al., Science 239*:487-491 (1988)).

### DNA sequencing

The *hipP* gene contained on plasmid pPX551 and the entire LKP1 operon contained on plasmid pHF1 were sequenced with standard M13 sequencing primers and with overlapping sense and antisense primers. All the DNA sequencing was done on an Applied Biosystems (ABI) 373A DNA Sequencer, utilizing the *Taq* thermal cycling DyeDeoxy™ Terminator sequencing kit from ABI, part # 901497. The LKP4 and LKP5 serotypes were sequenced directly from the PCR products using the PCR amplification primers and internal synthetic primers based on the LKP1 sequencing study.

### SDS-PAGE analysis

Sodium dodecyl sulfate polyacrylamide gel electrophoresis (SDS-PAGE) was performed in a 70 by 100 mm mini-gel system (Bio-Rad, Richmond, CA) using the method of Laemmli (Laemmli, U.K., *Nature (London) 227*:680-685 (1970)). Samples were reduced with β-mercaptoethanol or DTT in sample preparation buffer and boiled for 5 min. Gels were run at 150 V constant voltage. Separated proteins were detected by staining with Coomassie brilliant blue G-250 (Sigma).

### Partial purification of pili

LKP pili were purified according to previously described methods using differential pH solubility (Brinton, C.C., Jr. *et al., Pediatr. Infect. Dis. J. 8 Suppl*.:54-61 (1989)). Briefly, piliated bacteria were harvested from liquid culture by centrifugation and washed 2X in phosphate buffered saline, pH 7.2. The bacterial pellet was resuspended in 100 mM tris, pH 10.3, containing 150 mM NaCl at a ratio of 4 ml buffer/g wet weight of cells. Pili were sheared off of the cells by blending in an Oster miniblender for three 3 min bursts at 4°C. Bacterial debris was separated by centrifugation and discarded. The supernatant was dialyzed against 50 mM NaAcetate, pH 5.0 overnight to precipitate pili and denature other proteins. The pellet was collected by centrifugation at 15,000 x g at 4°C and dissolved overnight in 50 ml of 0.01 M CAPS buffer, pH 10.4 with gentle rocking. This cycle of acid precipitation and solubilization in basic buffer was repeated two more times. The final acid pellet was then resolubilized in 0.01 M NaPhosphate, pH 10.4 and non soluble material discarded. This soluble fraction was referred to as partially purified pili.

### Sequence of the LKP1 operon

The LKP1 operon was sequenced as described above and the full sequence is set forth in Table 5 (SEQ ID NO: 4). Sequence analysis identified six potential open reading frames (ORFs) in the LKP operon, including the *hipP* (at about nucleotide 1882-2532 of Table 5/SEQ ID NO: 4) and *hipC* (at about nucleotide 2854-3630 of Table 5/SEQ ID NO: 4) genes. All six ORF's in the LKP operon were identified as homologous to equivalent pilus operon genes in the pilus superfamily, as defined by multiple sequence alignment of proteins. Analysis of sequence alignment was also performed using Entrez Sequences Database Release 10.0 of the National Center for Biotechnology Information (National Library of Medicine, Bethesda, MD). Derived amino acid sequences of the ORFs are shown in Table 5 (SEQ ID NOs:5-10). A function for each reading frame was assigned based on sequence alignment analysis. There are five ORFs which appear to be grouped into an operon controlled by the *hipC* promoter region. After the *hipC* (periplasmic chaperon) gene, the second reading frame *hipR* (at about nucleotide 4016-6238 of Table 5/SEQ ID NO: 4) was designated, a membrane anchor protein the third ORF *hipM* (at about nucleotide 6259-6873 of Table 5/SEQ ID NO:4) was designated, a tip associated protein, (also referred to herein as a minor tip protein) and the fifth ORF *hipA* (at about nucleotide 6955-8265 of Table 5/SEQ ID NO: 4) was designated, a tip adhesin protein. The pilin gene (*hipP*) and the periplasmic chaperon gene (*hipC*) are transcribed in opposite orientations as in the LKP 4 operon with the promoter region having the previously identified TA repeats(van Ham, S.M. *et al., Cell 73*:1187-1196 (1993)). Since pHF1 expresses LKP1 pili in *E. coli,* there are 10 TA repeats in the intrapromoter region as described by van Ham *et al*.. These TA repeats are responsible for phase variation of the LKP pili phenotype, with loss of some of the repeats resulting in loss of piliation and a TA repeat number between 10 or 11 allowing expression of the LKP operon. As identified on the LKP1 operon was an ORF encoding an integrase (at about nucleotide 1495-1868 of Table 5/SEQ ID NO:4). Also located on the LKP1 operon was a sequence encoding an enzyme, peptidase (at about nucleotide 8395-9342 of Table 5/SEQ ID NO: 4).

The predicted size of the LKP1 *hipP* gene product is approximately 21.2 kilodaltons, assuming a signal sequence length of 20 amino acids, while the observed molecular weight in SDS-PAGE gels is approximately 27 kilodaltons. Part of this may be explained by the anomalous sequence migration of LKP pilins in general in SDS-PAGE gels (mature LKP4 migrates at a molecular size of 24 kilodaltons while its predicted size is 22.1 kilodaltons) but the exact explanation remains unknown.

### Sequence comparison of LKP serotypes 1, 4, and 5 hipP genes

This report represents the first sequence analysis of the *hipP* genes encoding LKP serotypes 1 and 5 (Figure 1). The *hipP* gene from an LKP4 expressing *Hib* strain has also been sequenced (van Ham, S.M. *et al*., *EMBO Jour. 8*:3535-3540 (1989)) and the derived amino acid sequence shows 99% identity with the LKP4 *hip*P derived amino acid sequence contained herein. The *hipP* gene sequences from *Hib* strains Eagan and M43 have been published (Forney, L.J. *et al*., *Infect. Immun. 59*:1991-1996 (1991)). The LKP1 *hipP* gene should encode a protein of approximately 21.5 kD while the predicted molecular weight of the LKP 4 *hipP* protein is 23.8 kD. The actual *hipP* gene products observed in recombinant *E. coli* are of approximately the correct sizes in Western blots for LKP4 and LKP5, but the LKP1 pilin runs aberrantly at a higher molecular weight than predicted at 26 kD. MacVector software was used to assess homology of these genes, with LKP4 *hipP* and LKP5 *hipP* proteins being 70 and 67% identical to LKP1 *hipP*, respectively. The alignment between the sequences is very good at the amino termini of the proteins, with three major areas of sequence divergence in the LKP1, 4, and 5 serotype genes farther into the proteins as shown in Figure 1. Since little cross reactivity is observed between anti-LKP1, anti-LKP4, or anti-LKP5 sera with intact pili of a heterologous serotype, the sequences responsible for the serotype specificity of the typing antisera must be located in these regions. By comparison of the sequences in GenBank to the LKP4 sequence, the *H. influenzae* type b M43 pilin (Gilsdorf, J.R. *et al*., *Infect. Immun. 58*:1065-1072 (1990)) sequenced by Gilsdorf *et al*. also appears to be an LKP4 serotype gene (data not shown).

### Example 2: Construction of LKP Type Pili-Producing E. coli Recombinants

### Bacterial strains

Piliated *Hflu* strains used for *E. coli* recombinant construction are LKP11/CB59, LKP10/88-0807 and LKP12/88-0677. Hemagglutination and serum agglutination were examined before making genomic library. *E. coli* strains XL1-Blue ^{MR} and HB101 were used as cloning host cell.

### DNA library construction and cosmid vector DNA

Chromosomal DNA from LKPI1, LKP10 and LKP12 were extracted and purified respectively by standard techniques. *Hflu* genomic DNA size is about 1.8 x 10⁶ bp. Chromosomal DNA was partially digested with restriction enzyme *Sau3A* I. Approximately 30kb DNA fragment was eluted from LMTA-gel (Sigma) and purified by phenol-chloroform method. The final DNA concentration is about 1µg/µl.

Vector DNA SuperCos I (Stratagene, La Jolla, CA) was digested with *Xba* I and dephosphorylated with calf intestinal alkaline phosphatase (CIAP). The *Xba* I and CIAP treated vector DNA was then digested with *Bam* HI restriction enzyme. About 6.5kb vector DNA fragment was obtained.

LKP11/CB59, LKP10/88-0807 and LKP12/88-0677 DNA fragments were ligated at the *Bam* HI site of the vector DNA SuperCos I, respectively. The ligated DNA was packaged into λ phage particles using Ciga-pack Gold kit (Stratagene, La Jolla, CA). The host cell for packaging was XL1-Blue^{MR}.

### Library screening

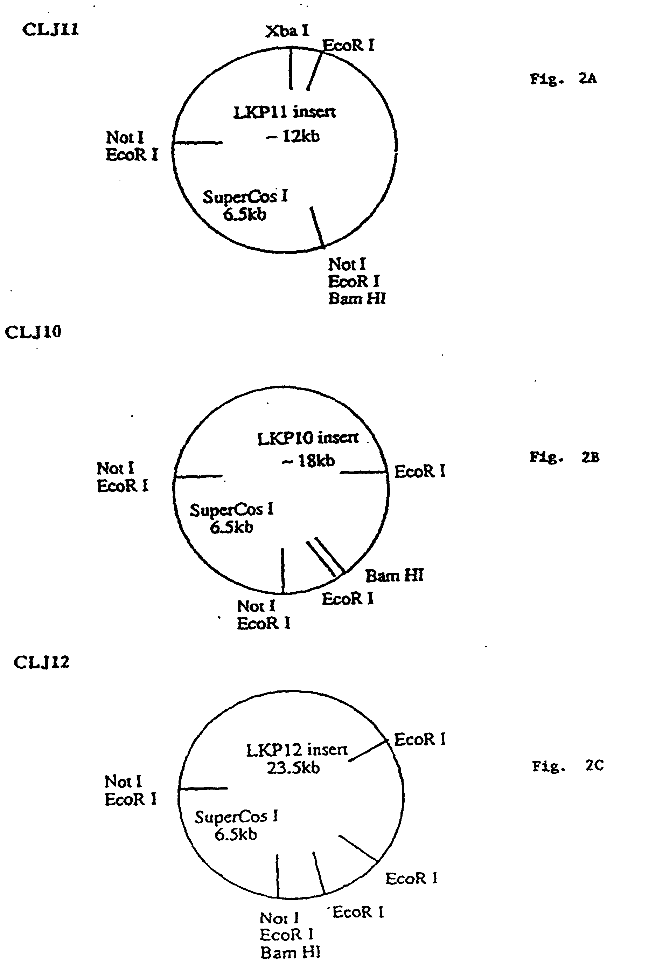

Recombinant expressed LKP type pili were screened by colony blot method. The concentration of anti-pilus sera from LKP11, LKP10 and LKP12 was 1:1000 dilution. The percentage of positive colony was 40/4200 for LKP11, 9/700 for LKP10 and 1/600 for LKP12. The cell piliation was examined by EM. The recombinants were verified by further HA and SA assay and they were named CLJ11 for LKP11, CLJ10 for LKP10 and CLJ12 for LKP12 (Figures 2A, 2B and 2C). Recombinants DNA was extracted and transformed to *E. coli* strain HB101 because XL1-Blue cell expresses type I pili. The recombinants DNA size is about 18.5kb for CLJ11. This was obtained by digestion and subsequent ligation using restriction site on insert and vector DNA. CLJ10 DNA is about 25kb and 35kb is for CLJ12. Partial DNA sequence is available for these recombinant inserts.

### Example 3: Protocols for the Purification of an LKP Pilus from an E.coli Recombinant Strain Using the Liquid Phase Method

### General Protocol

1. Inoculate recombinant *E. coli* cells in a 3 ml of LB media containing ampicillin and grow at 37°C until the OD 540nm reading reaches 0.6-0.8 (3-4 hours).
2. Transfer the cell suspension to 50 ml of medium and grow at 37°C until the reading at 540 nm reaches 0.8-1.0 (4-5 hours).
3. Transfer the 50 ml of cell suspension to 1L of medium in 2.8L flask and grow at 37°C overnight (16-18 hours) until a reading at 540 nm of 4.0-5.0 is obtained.
4. Harvest cells by centrifugation at 5000 rpm for 15 minutes.
5. resuspend the cells in 50 nM acetate buffer pH 5.0 and keep the suspension at room temperature for 1 hour.
6. Blend at 11000 rpm in large cup, or 14000 rpm in small cup, with omnimixer, ice for 3 minutes.
7. Titrate to pH 8.0 with 1 M HCl and let stand for 3 hours at room temperature.
8. Centrifuge at 12000 rpm for 20 minutes at 4°C. Weigh all pellets and discard.
9. Add 10 µl of DNase and RNase for each 100 ml of prep. Mix thoroughly and let stand for 10 minutes at room temperature.
10. Dialyze against several changes of 50 mM acetate buffer pH 5.0 overnight. If the prep does not reach pH 5.0 overnight, then dialyze longer against more changes of buffer.
11. Centrifuge at 16000 rpm for 60 minutes at 4°C to pellet the protein precipitant and pilus crystals.
12. Resuspend the pellet in about 25% original volume with 25 mM Tris-HCl buffer pH 8.0.
13. With gentle stirring add Triton X-100 and EDTA to the prep to yield final concentration of 0.2% and 5 mM. Stir gently overnight at 4°C.
14. Clarify the prep by centrifuging at 16000 rpm for 60 minutes at 4°C.
15. Add NaCl and PEG 8000 to final concentration of 0.5 M and 3.0% respectively then incubate and prep over ice for 2 hours.
16. Centrifuge the prep at 16000 rpm for 60 minutes at 4°C to pellet the pilus crystals.
17. Resuspend pellet in 25 mM Tris-HCl pH 8.0 in 1/3 of previous volume. Use less solution a lesser yield of pilus crystals is obtained.
18. Repeat steps 13 to 17.
19. Resuspend pellet in 25 mM Tris-HCl pH 8.0. Depending on purity and amount of material alternative solubilization and crystallization steps may be continued as needed.

During purification, sample after each step and use SDS-PAGE tor examine purity of the samples. Dark field microscopy assay is needed in assistance for purity checking. It is necessary to use UV scanning to determine any contamination by DNA or RNA.

Since Triton X-100 has a strong absorbance at 280 nm, it is important to remove the residual Triton X-100 by crystallization, one time, or more, of pili by PEG and NaCl after purification. This avoids false readings at 280 nm when one determines concentration of pilus preparations by UV method.

### Purification of LKP 5 Pili

1. Harvest in 80 mM PBS pH 5.0 using 5-10 ml/tray.
2. Titrate prep to pH 5.0 with 6N HC1 if necessary.
3. Blend with omnimixer over ice for 3 minutes (average speed=9800 rpm) (up to 11000 rpm if possible in larger cups and up to 14000 rpm in small cups).
4. Titrate to pH 9.0 with 5 M NaOH and let stand for 3 hours at room temperature. It may be necessary to stir gently to prevent pH changes. Monitor pH throughout and adjust if needed. (If cultures were grown in broth, then titrate with a 1 M solution of buffer (Tris) instead of NaOH.)
5. Centrifuge at 15300 g for 20 minutes at 4°C. Transfer supernatant to clean bottles and clarify a second time as before. Weigh all pellets and discard.
6. Adjust pH of supernatant to 8.0 and add 10 ul of DNase and RNase for each 100 ml of prep. Mix thoroughly and let stand for 10 minutes at room temperature.
7. Dialyze against several changes of 40 mM acetate buffer pH 5.0 overnight. If prep does not reach pH 5.0 overnight then dialyze longer against more changes of buffer.
8. Centrifuge at 18600 g for 60 minutes at 4°C to pellet the pilus crystals (crystals not typical for clear pili).
9. Resuspend the pellet in about 25% original volume with 25 mM Tris-HCl pH 9.0 using rubber policeman. Stir gently at 4°C (avoid forming) several hours. Break up large pieces with gentle pipeting as needed.
10. With gentle stirring, add Triton X-100 (2% stock) to the prep to yield a final concentration of 0.4% and add EDTA (25 mM stock) to a final concentration of 5 mM. Incubate overnight at 4°C.
11. Clarify the prep by centrifuging at 186000 g for 60 minutes at 4°C. Transfer supernatant to clean flask.
12. Adjust the pH of the supernatant to below 8.0 using 1 N HCl.
13. Add NaCl (5 M stock) to a final concentration of 0.5 M and PEG (30% stock) to final concentration of 3% then incubate the prep over ice for 0.5 hour. Inspect in darkfield for crystals. Increase time if needed but it is critical not to overexpose pili to PEG because resolubilization becomes increasingly difficult with increasing times.
14. Centrifuge prep at 18600 g for 60 minutes at 4°C to pellet the pilus crystals.
15. Wash pellet with 40 mM citrate buffer pH 5.0 to remove excess PEG/NaCl. Then centrifuge at 186000 g for 60 minutes (2 times).
16. Resuspend pellet in 25 mM Tris-HCl pH 9.0 in 1/3 to 1/2 previous volume. Solubilize by swirling followed by gentle pipetting. Run sample on a gel to check for purity. If necessary, continue with step 17.
17. Add Triton x-100 to the prep to yield a final concentration of 0.4% and add EDTA to a final concentration of 5 mM then incubate overnight at 4°C (see step 10 for details).
18. Adjust the pH of the prep to below 8.0 using HCl (between 7 and 8).
19. Add NaCl to a final concentration of 0.5 M and PEG to a final concentration of 3% then incubate the prep over ice for 0.5 hours (see step 13 for details).
20. Centrifuge prep at 186000 g for 60 minutes at 5°C to pellet pilus crystals.
21. Resuspend the pellet in 25 mM Tris-HCl pH 9.0 to solubilize pili (see step 16 for details). Check for purity by SDS-PAGE. If necessary, continue with step 22.
22. Add Triton X-100 to the prep to yield a final concentration of 0.4% and add EDTA to a final concentration of 5 mM then incubate overnight at 4°C (see step 10 for details).
23. Clarify by centrifuging at 18600 g for 60 minutes at 4°C.
24. Add NaCl to a final concentration of 0.5 M and PEG to a final concentration of 3M then incubate the prep over ice for 0.5 hour (see step 13 for details).
25. Centrifuge at 18600 g for 1 hour at 4°C. Discard supernatant.
26. Resuspend pellet in Tris-HCl pH 9.0. Depending on amount and purity of material, alternating solubilization/crystallization steps may be continued as needed.

During purification process, monitor pellet material and supernatant by darkfield and/or gel and/or scan. May need to reprocess.

Purity by SDS-PAGE check: Repeat Triton step as needed, but avoid SDS reaction steps in previous protocols because of high losses of pili.

### Example 4 : Purification of LKP pili by HPLC and other Column Methods

Besides detergent extraction and PEG precipitation, LKP pili also can be purified by HPLC, FPLC and other column methods. These methods are good particularly for unknown LKP pili. Normally, pili are partially purified by extraction and precipitation first until the pilus solution is clear, concentrated and very small in size. If the preparation still is not pure as determined by SDS-PAGE, column methods would be the application of the choice. Sizing columns are preferred to be used for this purpose. Prior to loading to a column, treatment for further purification of the pilus sample is important. The detergent used for partial purification of pili should be removed from pilus samples by dialysis or other known techniques. Detergent significantly reduces column separation resolution. Size exclusive column requires a small sample volume.

For HPLC or FPLC, the loading volume of 50 µl to 200 µl is recommended, and for other routine LC gel filtration columns, the sample loading volume depends on the length and size of the column. A 1 ml of pilus sample is preferred for a column with a total volume of 50 ml. Since pili have a low absorbance at 280 nm, a higher sensitivity for monitor is recommended. Available protein eluted from column can be monitored at 230 nm. Further purification of proteins can be performed by HPLC. Column methods of purification are also useful for isolation of pilin from pili.

### Example:5 Protocol for the Purification of an LKP Pilus from an Hflu Stratin or E. coli Recombinant Strain Using Solid Phase Method

Generally speaking, recombinant strain expresses pilus structural protein better than parent strain, H*flu* , does, therefore, it is easier to purify pili from the recombinant cells. However, due to the fact that the *E. coli* recombinant strain expresses the pilus protein as same as the parent *Hflu* does,purification procedures of pilus rods from *Hflu* or from recombinant strain are basically the same. Growth of *Hflu* strain requires chocolate agar media and certain CO₂ and humidity. Growth of *E. coli* recombinant strain needs LB agar media containing ampicillin.
1. Harvest in 80 mM PBS pH 5.0 using 5 ml/tray. Use a smoothed glass edge to scrape wet cells and then transfer the cell suspension to omnimixer cup. If cells are made surface only use media surface moisture to collect wet cells.
2. Titrate prep to pH 5.0 with 2 M acetate buffer if necessary.
3. Blend at 14000 rpm with omnimixer over ice for 3-5 minutes.
4. Titrate to pH 8.0 with 1 M Tris-HCl buffer and monitor pH change by pH meter. It may titrate to pH with 2.5 or 5 M NaOH instead of Tris buffer, if prep contains a lot of wet cells. Be careful to avoid lysis of cells when use NaOH. Incubate the prep at room temperature for 3 hours.
5. Centrifuge at 12000 rpm for 20-30 minutes at 4°C. Weigh all pellets and discard.
6. Add 10 µl of DNase and RNase for each 100 ml of prep. Mix thoroughly and let stand for 10 minutes at room temperature.
7. Dialyze against several changes of 50 mM acetate buffer, pH 5.0, overnight. If the prep does not reach pH 5.0 overnight, then dialyze longer against more changes of buffer.
8. Centrifuge at 16000 rpm for 60 minutes at 4°C to pellet protein precipitate and pilus crystals.
9. Resuspend pellet in about 25% original volume with 25 mM Tris-HCl buffer, pH 8.0.
10. With gentle stirring, add Triton X-100 and EDTA to prep to yield final concentration of 0.2% and 5 mM. Stir gently overnight at 4°C.
11. Clarify prep by centrifugation at 16000 rpm for 60 minutes at 4°C.
12. Add NaCl and PEG 8000 to final concentration of 0.5 M and 3.0%, respectively, then incubate the prep over ice for 2 hours. LKP pili with different length and dimer may be crystallized in different concentrations of NaCl and PEG 8000. Therefore a concentration test for NaCl and PEG to crystallize different pili is important.
13. Centrifugeat 16000 rpm for 60 minutes at 4°C to pellet pilus crystals.
14. Resuspend pellet in 25 mM Tris-HCl, pH 8.0 in 1/3 previous. Use even less solution if a smaller yield of pilus crystal is found.
15. Repeat from step 10 to step 14.
16. Resuspend pellet in 25 mM Tris-HCl, pH 8.0. Depending on purity and amount of material, alternate solubilization and crystallization steps may be continued as needed.

During purification sample after each step and use SDS-PAGE to examine purity of the samples. Dark field microscopy assay is needed in assistance for purity checking. It is necessary to use UV scanning for finding out any contamination by DNA or RNA.

Since Triton X-100 has a strong absorbance at 280 nm it is wise to remove the residual of the detergent by one more time crystallization of pili by PEG and NaCl after purification This avoids false readings at 280 nm when one determines concentration of pilus preparation by UV method.

### Example 6: Construction of MBP-Δ3'Tip Fusion Protein

The genetic fusion was constructed by using PCR primers to obtain a portion of the LKP1 tip gene from pHFl which would be in frame with the MBP protein gene in the vector pMAL-p2. The primers were designed so that the carboxyl terminal of approximately 100 amino acids of the tip protein would be deleted and replaced with a stop codon. The amino terminal portion of the protein was PCR'd in frame with an appropriate restriction site at the approximate point of the signal sequence cleavage site which was determined by analogy to other bacterial signal sequences and the hydrophobicity profile of the deduced amino acid sequence of the tip protein. The amino acid sequence of the fusion protein is shown in Figure 2. The partial sequence of the LKP tip protein of the fusion protein is underlined.

### Expression of the fusion, purification and antisera production

The protein was expressed in *E. coli* BL21 (an *onnipT.lon* K-12 strain) grown in SOB broth containing ampicillin at 100 µg/ml at 28 C after induction with 0.2 mM IPTG. The cells were pelleted by centrifugation and washed 1 time in PBS. The cells were resuspended in 20 mM Tris, pH 7.5 containing 2 mM EDTA and 400 mM NaCl at a ratio of 20 ml/liter of original culture. The cells were lysed by passing through a French pressure cell 3 times and the cell debris removed by low speed centrifugation at 8 times x g for 20 minutes at 4°C. The supernatant was diluted 5-fold in the same buffer used for breakage and passed over a 15 ml bed volume amylose resin column at 1 ml/min at room temperature. After the lysate was run over the column, the column was washed with 15 bed volumes of the lysing buffer at 5 ml/min. The bound material was eluted using washing buffer containing 10 mM maltose. The elution was done with 50 ml of buffer at 1 ml/min and the eluant pooled. The resulting protein mixture was analyzed by SDS-PAGE and Western Blot and anti-MBP sera and found to contain the fusion, breakdown products, and full length MBP. Little other material was detected.

The fusion proteins, MBP and breakdown products eluted as a complex. Mice were immunized with 10 µg doses of the complex using 100 µg MPL as adjuvant.
Immunizations were done subcutaneously at weeks 0, 4, and 6 and the mice exsanguinated on week 8. The negative control sera was mouse anti-MBP sera made against purified MBP using the same purification and immunization protocols.

### Ant i -GST sera

The GST fusion was constructed using the complete LKP tip gene, including the signal sequence. The gene was PCR'd out from PHF1 with the appropriate restriction enzyme sites for insertion into pGEX-3X in frame, and expressed in *E. coli* DH5α. The cells were grown in SOB containing 100 µg/ml ampicillin and induced with IPTG at 0.2 mM at 37°C for 2 hours. The cells were harvested and washed in PBS, then resuspended in PBS and lysed by passing through a French pressure cell. Cell debris was harvested by centrifugation and washed 3 times with buffer containing 1% Triton X-Zwittergent 3-14 and the inclusion bodies recovered by centrifugation. The inclusion bodies were solubilized in 5 M guanidine HCl and analyzed by SDS-PAGE. The guanidine concentration was lowered to 2.5 M by dialysis and the soluble inclusion bodies stored at 4°C. The antisera was made by running preparative 10% SDS-PAGE gels and cutting the fusion band out of the gel. The acrylamide-protein band was minced using a scalpel and mixed with MPL (100 µg) and injected into mice 3 times at weeks 0, 4, and 6. Mice were bled at week 8.

### Example 7: Removal, Purification and Identification of H. influenzae LKP Pilus Tip Adhesin Protein

This is the first demonstration that tip adhesin protein from *H. influenzae* LKP1 pili can be removed without depolymerization of pilus rods. Free tip adhesin protein can be isolated and purified by means of dialysis and prep-electrophoresis. Purified tip adhesin can be identified by the antiserum from a constructed genetic fusion protein, which is from a portion of LKP1 tip gene and MBP (maltose binding protein) gene, using Western blot analysis. Specific binding was detected between the purified tip protein and fusion protein antiserum, which clearly shows that the protein purified from LKP1 pilus prep is LKP1 tip adhesin protein.

Activity assays with human red blood cell (RBC) ghosts demonstrated that purified tip protein binds to a native ghosts preparation but not does not bind to denatured RBC ghosts, indicating that puri fied tip protein is biologically functional or at least partially functional.

### Removal of Tin Protein from Pilus Rods

1. Dialyze purified LKP1 pili in 200 mM Gly-HCl buffer, pH 2.0 containing 5 M NaCl, at room temperature for 4 to 6 hours.
2. Transfer the dialysis bag into a 25 mM Tris-HCl buffer, pH 8.0 and dialyze for several hours till the pH of pilus prep reaches to pH 8.0.
3. Add SDS to the pilus prep to a final concentration of 0.1% and incubate in 4°C for 10 hours.
4. Dialyze the pilus prep in 50 mM citrate buffer, pH 5.0 overnight.
5. Pilus aggregates can be removed by centrifugation and most free tip protein is retained in the supernatant.

Tip protein can be completely removed by 2% SDS in 25 mM Tris buffer without depolymerization of pilus rods, but the SDS may damage the activity of the protein. 0.1% SDS only removes about 20-30% of total tip protein, however, the protein maintains biological activity. The results also demonstrated that 4M urea and 2M GuHCl in pH 2.0 buffer can partially remove tip protein from pilus rods without depolymerization.

### Purification of tin protein

1. Mix concentrated tip protein with SDS-PAGE sample treatment buffer without the SDS and β-mercaptolethanol. The ratio is 2.5 ml of pilus prep to 0.3 ml of sample treatment buffer.
2. Load the sample to a 12% SDS-PAGE (0.1% SDS) in Prep-Cell (Bio-Rad) with the length of stacking gel of 0.8-1.0 cm and running gel of 5cm.
3. Run the gel at 300 volt with cooling system for 6-8 hours, and monitor the elution at 280 nm.
4. Pool the fractions containing tip protein and concentrate.
5. Determine the purity of the pooled fractions by mini-SDS-PAGE. The identification of purified tip protein by anti-KLP1-MBP fusion protein is shown in Figure 4. The binding activity of purified tip protein with human red cell ghosts is shown in Figures 5 and 6. Figure 7 compares adhesin proteins from different LKP type pili by SDS/PAGE.

### Example 8: Serotype Analysis

The *Haemophilus influenzae (Hflu)* bacterioplex is a differentiated complex of bacterial phases, or cell types, socially organized to facilitate the protein appendages expressed on the surface of *Hflu*, and also secreted from *Hflu* in free form, carrying specific adhesion determinants for binding to human cell membrane receptors. Pili adapt pathogenic bacteria to life in vertebrate hosts by mimicking the functions of the host's own proteins. Pilus functions include attaching bacteria to a variety of host cells and tissues and stimulating the host's immune system in ways which benefit the bacteria and damage the host. Pili are transmission, virulence, dissemination, pathogenicity and immunity factors in most bacterial diseases.

The expression of pili is controlled by a genetic switching mechanism, phase variation, in which pilus expression and pilus type are switched on and off at probabilities which vary with and are determined by conditions and signals in the immediate environment of the bacteria. Under some conditions the switching probabilities can be very high, as high as 10⁻² per bacterial cell division. Under other environmental conditions the probability of the same phase switch can be 10⁻⁶ or lower. Phase switching is accompanied by both reversible and irreversible rearrangements in the DNA of pilus operons. Phase switching during in vitro growth is frequently accompanied by deletions to pilus operon genes such that nonpiliated phases remain irreversibly in that phase.

By purifying *Hflu* pili from different isolates and producing antisera to the purified preparations distinct LKP pilus serotypes have so far been identified. The expression of the different serotypes is used as a marker to identify the different piliation phases of the *Hflu* bacterioplex.

**TABLE 1**

| | | L=1 | L=2 | L=3 | D=3 | D=4 |
|---|---|---|---|---|---|---|
| LKP1 | N=4 | 0 | 0 | 4 | 0 | 4 |
| LKP2 | N-2 | 0 | 1 | 1 | 0 | 2 |
| LKP3 | N=0 | 0 | 0 | 0 | 0 | 0 |
| LKP4 | N=1 | 0 | 1 | 0 | 0 | 1 |
| LKP5 | N=5 | 0 | 1 | 4 | 0 | 5 |
| LKP6 | N=12 | 0 | 2 | 8 | 1 | 9 |
| LKP7 | N=3 | 0 | 0 | 2 | 0 | 2 |
| LKP8 | N=0 | 0 | 0 | 0 | 0 | 0 |
| LKP9 | N=0 | 0 | 0 | 0 | 0 | 0 |
| LKP10 | N=26 | 1 | 8 | 17 | 2 | 24 |
| LKP11 | N=22 | 0 | 6 | 16 | 0 | 22 |
| LKP12 | N=12 | 0 | 3 | 7 | 2 | 8 |
| LKP13 | N=0 | 0 | 0 | 0 | 0 | 0 |
| LKP14 | N=9 | 1 | 2 | 6 | 1 | 8 |
| LKP15 | N=6 | 0 | 5 | 1 | 0 | 6 |
| LKP16 | N=9 | 0 | 4 | 5 | 3 | 6 |
| LKP17 | N=17 | 0 | 6 | 11 | 2 | 15 |
| LKP18 | N=12 | 1 | 4 | 7 | 1 | 11 |
| LKP19 | N=3 | 0 | 1 | 3 | 0 | 3 |
| LKP20 | N=15 | 1 | 6 | 8 | 3 | 12 |
| Total Strains = 77 | | 4 | 50 | 99 | 15 | 136 |

| | | | | | | |
|---|---|---|---|---|---|---|
| L=1 is length <0.2µ | | | | | | |
| L=2 is length <0.2µ<0.5µ | | | | | | |
| L=3 is length <0.5µ | | | | | | |
| D=3 is 3nm diameter ("thin") | | | | | | |
| D=4 is 4nm diameter ("thick") | | | | | | |

The frequency of each LKP serotype was determined for all serotypable cultures and for all cultures expressing by counting types on both single expressors and multiple expressors. Sixteen of the 20 serotypes were found on typically LKP piliated cultures and 90% of these cultures were serotypable in the 20-type system. The frequency distribution of serotypes for these cultures is shown in Table 1.

Three different LKP pilus operon genes were selected, the pilin gene, anchor gene and adhesin gene, which had all exhibited sequence similarity among different serotypes in multiple sequence alignments, but were also characteristic of *Hflu* LKP pili. Sequences were selected from these genes that would serve as suitable primer sequences flanking each gene for use in a PCR reaction.
All three pairs of primers were synthesized and used in a PCR reaction to amplify segments of DNA extracted from *Hflu* isolates.

Data showing the presence of LKP pilus operons in tested *Haemophilus influenzae* strains is shown in Table 2.

| TABLE 2 | | | | | | |
|---|---|---|---|---|---|---|
| CORRELATION BETWEEN THE PRESENCE OF LKP PILUS OPERON GENETIC MATERIAL IN *H. INFLUENZAE* ISOLATES AND THE EXPRESSED LKP PARAMETERS OF PILIATION AND HEMAGGLUTINATION | | | | | | |
| LKP Parameter | Total | PCR Done | PCR + | PCR - | Fraction PCR + | Percent PCR + |
| Pilus Length 0 | 74 | 68 | 59 | 9 | 59/68 | 87% |
| Pilus length 3 | 101 | 93 | 82 | 11 | 82/93 | 88% |
| HA+ | 148 | 139 | 115 | 24 | 115/139 | 83% |
| HA- | 166 | 149 | 119 | 30 | 119/149 | 80% |
| Pilus diam. 3 | 40 | 38 | 28 | 10 | 28/38 | 74% |
| Pilus diam. 4 | 172 | 159 | 136 | 23 | 136/159 | 86% |
| Serotypable | 189 | 173 | 149 | 24 | 149/173 | 86% |
| Not serotypable | 54 | 63 | 53 | 10 | 53/63 | 84% |

| | | | | | | |
|---|---|---|---|---|---|---|
| 1. Pilus length 0 means nonpiliated. | | | | | | |
| 2. length 3 means >0.5 microns (longest, typical of LKP pili). | | | | | | |
| 3. HA+ means positive for hemagglutination of human red blood cells; typical of LKP pili. (These isolates are not recalcitrant by definition.) | | | | | | |
| 4. HA- means negative for hemagglutination of human red blood cells; typical of SNN pili. (These isolates are recalcitrant since all isolates were hemadsorbed at least once.) | | | | | | |
| 5. Pilus diameter 3 means the isolates express pili with diameters typical of SNN pili. | | | | | | |
| 6. Pilus diameter 4 means the isolates express pili with diameters typical of LKP pili. | | | | | | |
| 7. Serotypable means the isolates agglutinate under standard conditions with at least one of the LKP pilus typing antisera in the 1-20 system. | | | | | | |
| 8. Not serotypable means the isolates do not agglutinate with any of the LKP pilus typing antisera in the 1-20 system. | | | | | | |

### Example 9: Hybridization Assay for Haemophilus Influenzae Assay Probe Construction

An approximately 1100 bp fragment from plasmid pHF1 (Karasic, R. *et al., Pediatr. Infect. Dis . J. 8 (Suppl.)*:S62-65 (1988)) which contains the LKP1 serotype operon was amplified by PCR using primers which hybridize at the 5' and 3' ends of the *hip*A gene. This gene encodes the tip adhesin protein of the LKP1 pili. The PCR reaction included digoxigenin labeled dUTP along with the four dNTPs to label the PCR reaction product with digoxigenin. This probe was electrophoresed on an agarose gel and purified by cutting out the ~1.2 kb band and extracting the DNA by standard methods. The probe was redissolved in 30 µl of appropriate buffer.

### Hybridization Assay

Eleven randomly chosen Haemophilus influenzae clinical isolates were grown on BHI-XV plates at 37°C with 5% CO₂ and also streaked onto BHI agar. All isolates grew only on the BHI-XV plate, indicating that they were *H. influenzae*. The isolates included 2 *Hib* strains and 9 *NTHi*. The strains were inoculated onto a nylon membrane placed onto BHI-XV agar. Five clinical isolates of another respiratory pathogen, *Moraxella catarrhalis* were also spotted onto the filter. The bacteria were grown overnight at 37°C in 5% CO₂. After growth, 2 *Bordetella pertussis* strains were spotted onto the filter. Filters were processed for colony hybridization according to the method of Maniatis *et al.* (*Molecular Cloning: A Laboratory Manual,* 1991, Cold Spring Harbor Laboratories, Cold Spring Harbor, NY). Filters were blocked in pre-hybridization solution as described by Boehringer-Mannheim for the Genius™ system at 65°C for 3 hours. Colony debris was removed by gentle rubbing with wet paper towels. The probe, 30 µl, was added to 5 ml of pre-hybridization solution and boiled for 10 minutes to denature the DNA. Probe was immediately added to the filter and allowed to hybridize overnight at 65°C. Filters were washed in 2X SSC, 0.1% SDS, 2X for 5 min/wash at room temperature followed by 2, 15 minute washes with 0.2X SSC, 0.1% SDS at 65°C. Bound probe was detected using alkaline phosphatase labeled anti-digoxigenin antibodies as described by the manufacturer. Results are shown in Table 3.

**Table 3 HYBRIDIZATION OF dig-LABELED LKP 1 TIP PROBE TO RANDOM CLINICAL ISOLATES**

| Number of Positive Results | | | | |
|---|---|---|---|---|
| Bacterial Strain | Strong Signal | Weak Signal | No Signal | # Total |
| *H. influenzae* | *4* | 4 | 3 | 11 |
| *M. catarrhalis* | 0 | 0 | 5 | 5 |
| *B. pertussis* | 0 | 0 | 0 | 2 |

The probe was specific for *H. influenzae* with no hybridization seen with either *M. catarrhalis* or *B. pertussis*.

### Hybridization Assay of Nontypable Strains of Haemophilus influenza pili

Ten LKP pili expressing NTHi strains which express differing serotypes of LKP pili, along with *Hib* Eagan were grown on a nylon filter overlayed onto chocolate agar at 37°C in 5% CO₂. An additional *NTHi* isolate was also included. After growth, two strains appeared yellow on the filter which was suggestive of non-*Haemophilus* bacteria, so they were tested by growth on BHI and BHI-XV. This experiment showed them to be contaminants and not *NTHi*. The filter was removed from the agar and processed as described above. The probe from the first experiment was reboiled and added to the filter as before, except that the hybridization temperature was lowered to 62°C. The filter was washed as before except that the wash temperature was also 62°C. Bound probe was detected as above. Results are shown in Table 4.

**Table 4. HYBRIDIZATION OF dig-LABELED TKP TIP PROBE TO LKP TYPE STRAINS**

| LKP Serotype | Signal with probe | No signal with probe | ID of strain |
|---|---|---|---|
| 5 | Strong | | NTHi |
| 2 | Moderate | | NTHi |
| 9 | Strong | | NTHi |
| 1 | Strong | | NTHi |
| 6 | Moderate | | NTHi |
| 13 | Strong | | NTHi |
| 4 | Strong | | NTHi |
| 7 | Moderate | | NTHi |
| | | X | Contaminant |
| | | X | Contaminant |
| 10 | Weak | | NTHi |
| 4 | Strong | | Hib |

The results set forth above establish that the DNA probes hybridized selectively to Haemophilus influenzae.

Those skilled in the art will recognize, or be able to ascertain using no more than routine experimentation, many equivalents to the specific embodiments described herein.

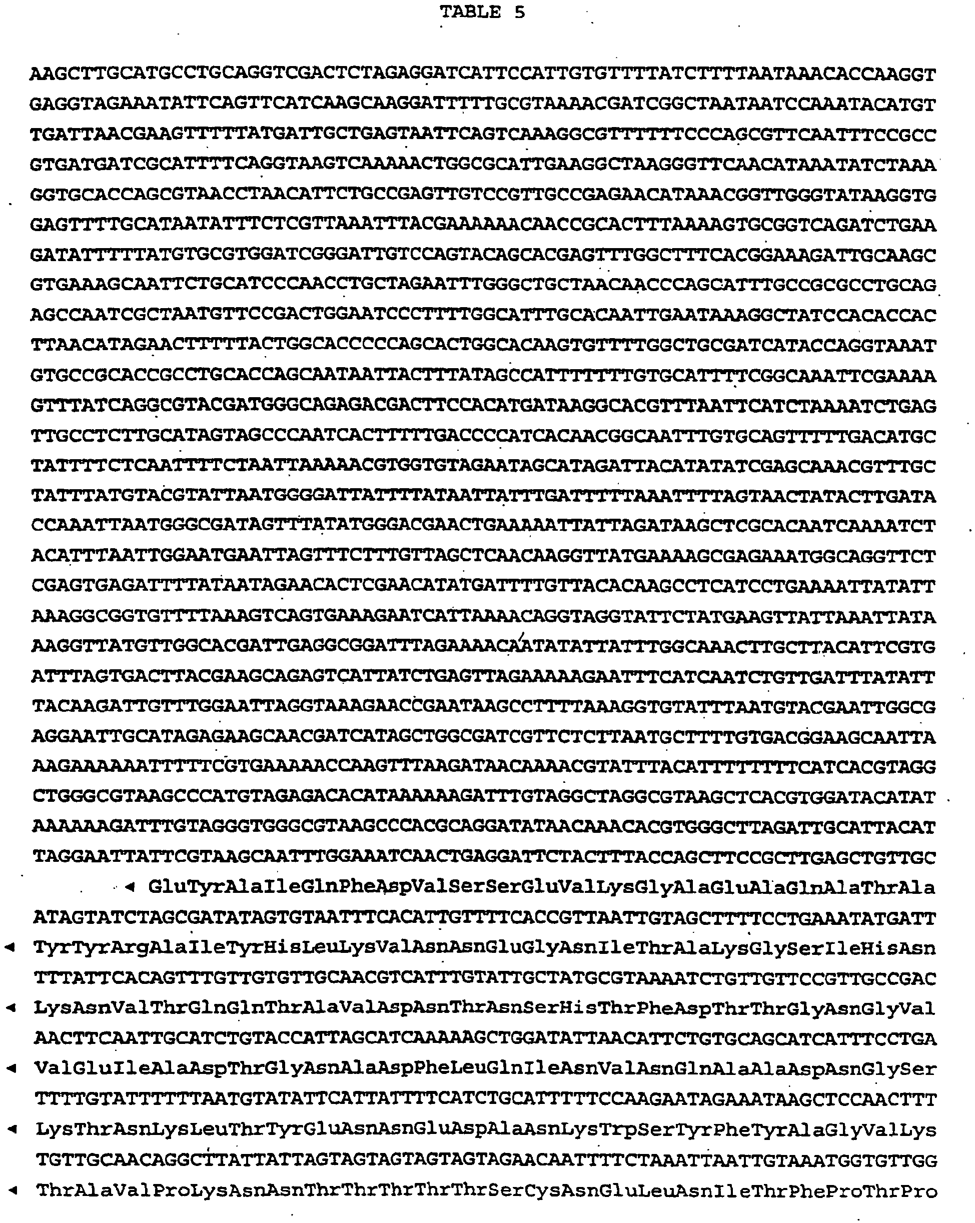

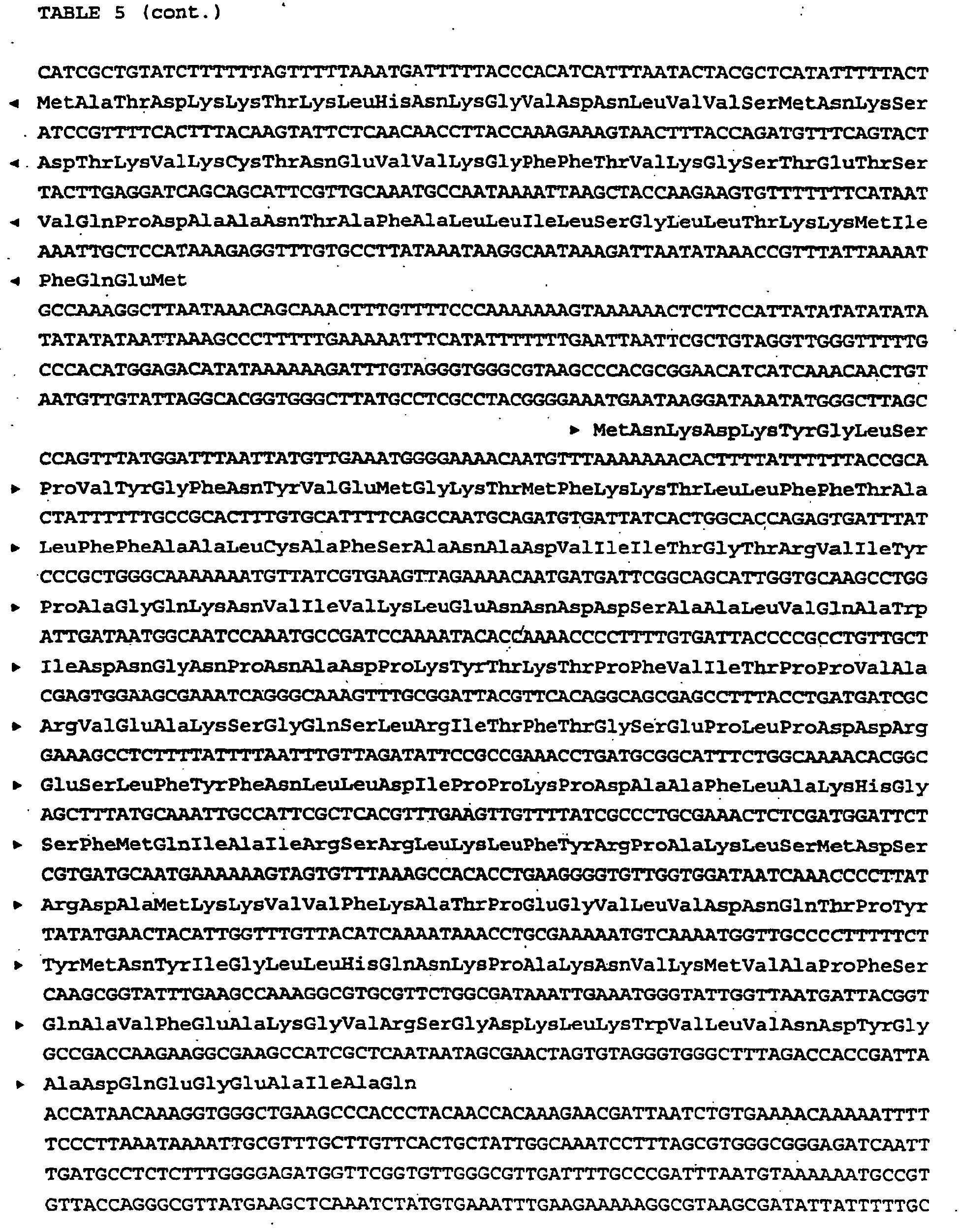

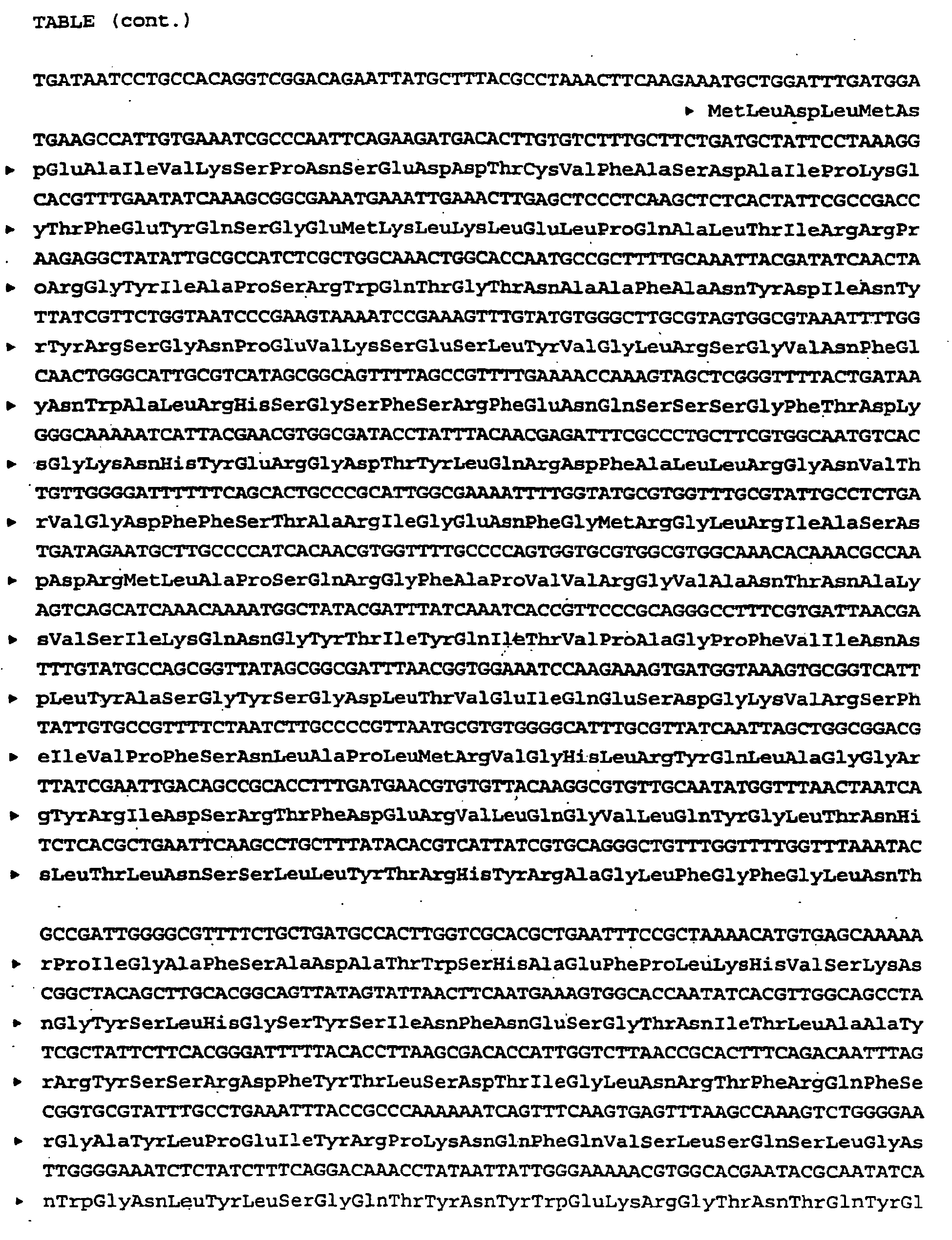

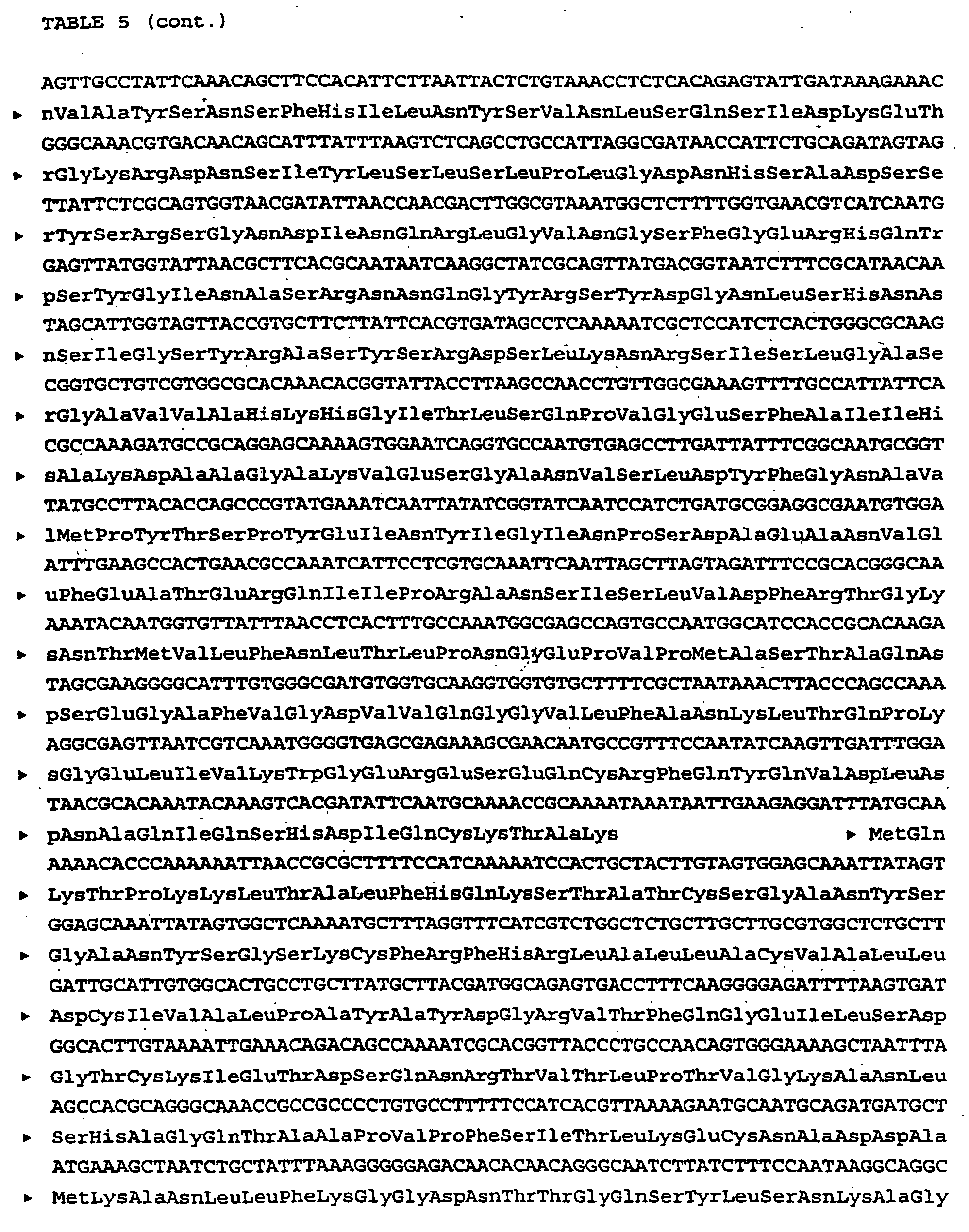

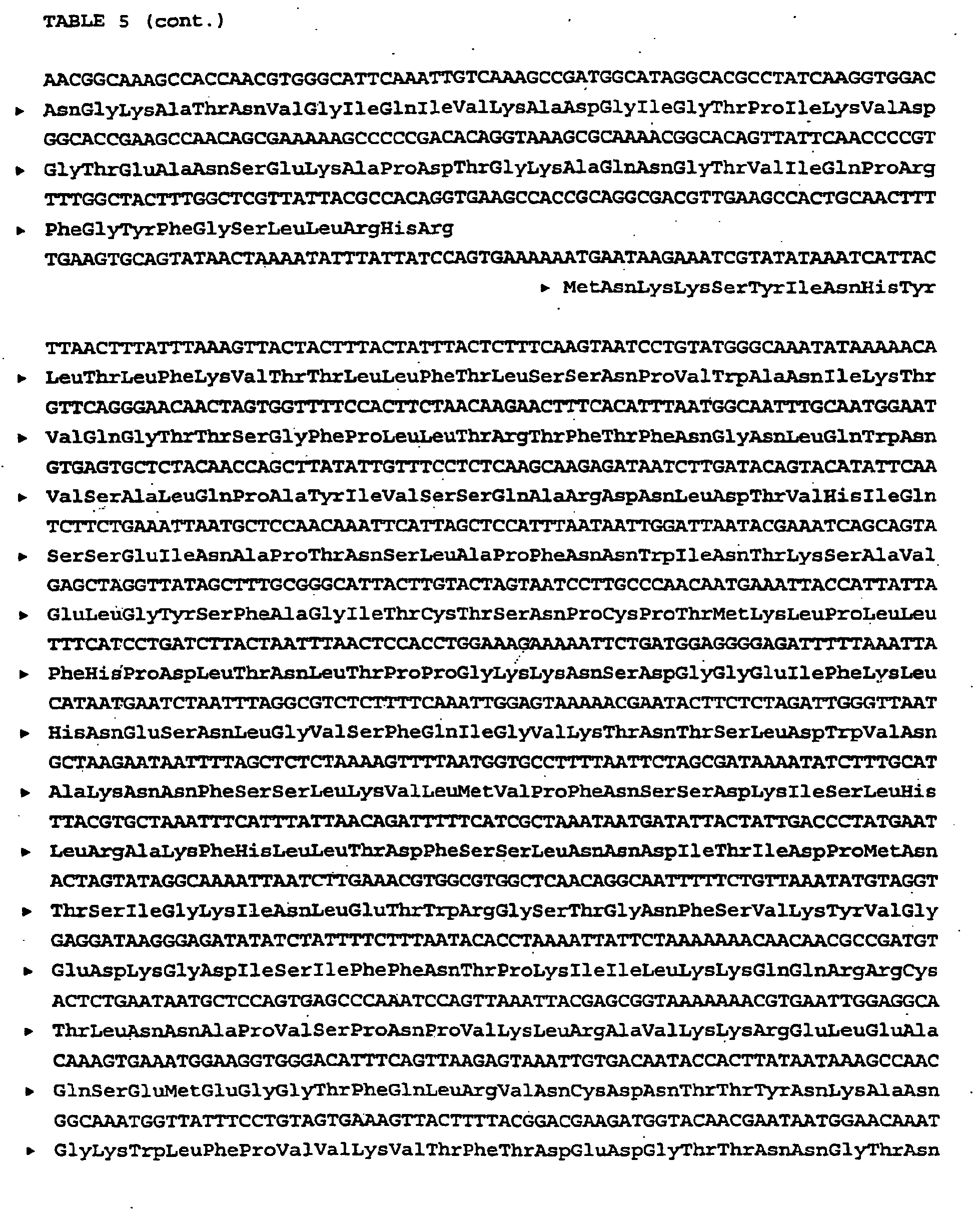

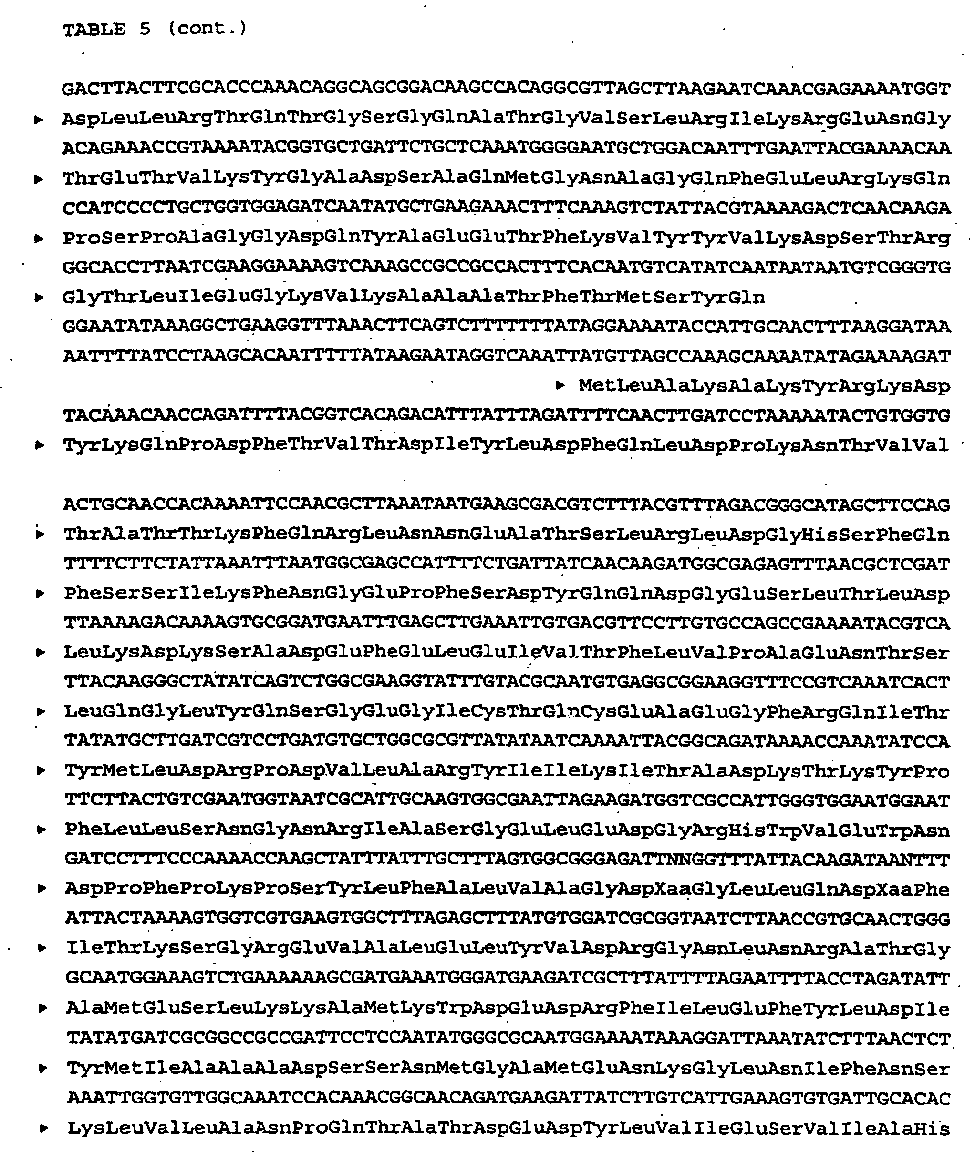

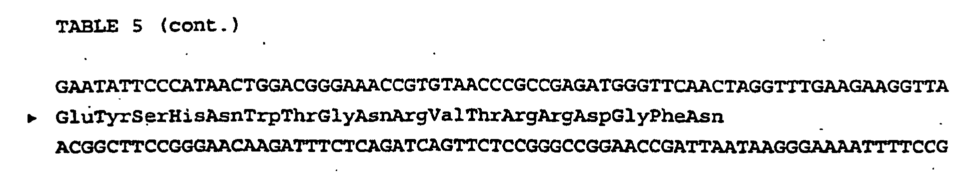

## Claims

1. An isolated nucleic acid sequence selected from the group consisting of:
(i) an isolated nucleic acid sequence selected from the group consisting of:
a) nucleotides 6955 to 8265 of Table 5;
b) nucleic acid sequences that hybridize to the nucleotides of a) under conditions of high stringency; and
c) RNA sequences transcribed from the nucleotides of a), or b); and
(ii) an isolated DNA sequence which hybridizes to a DNA sequence encoding nontypable Haemophilus influenzae serotype 1 LKP tip adhesin protein under conditions of high stringency, wherein the DNA sequence encoding the serotype 1 LKP tip adhesin protein consists of nucleotides 6955 to 8265 of Table 5.

2. An isolated nontypable Haemophilus influenzae protein, comprising:
a serotype 1 LKP tip adhesin protein having the amino acid sequence encoded by the ORF of nucleotides 6955 to 8265 of Table 5, and biologically active fragments thereof which bind to native red blood cell ghost preparations but do not bind to denatured red blood cell ghosts.

3. A recombinant expression vector comprising a DNA insert, wherein said DNA insert hybridizes to the complement of a nucleotide sequence encoding nontypable Haemophilus influenzae LKP tip adhesin protein under conditions of high stringency, said nucleotide sequence consisting of nucleotides 6955 to 8265 of Table 5, wherein said expression vector expresses a nontypable Haemophilus influenzae tip adhesin protein in a procaryotic or eucaryotic cell.

4. The recombinant expression vector of Claim 3 wherein the DNA insert encodes a protein comprising the amino acid sequence encoded by the ORF of nucleotides 6955 to 8265 of Table 5.

5. A procaryotic or eucaryotic host cell transformed with an expression vector of Claim 3.

6. An in vitro method of producing nontypable *Haemophilus influenzae* protein of Claim 2 using the recombinant expression vector of Claim 3 in a procaryotic or eucaryotic host cell comprising transfecting the host cell with the recombinant expression vector and maintaining the transfected host cell under conditions suitable for the expression of nontypable *Haemophilus influenzae* LKP pilus proteins in the host cell.

## Patentansprüche

1. Isolierte Nucleinsäuresequenz, ausgewählt aus der Gruppe bestehend aus:
(i) einer isolierten Nucleinsäuresequenz, ausgewählt aus der Gruppe bestehend aus:
a) Nucleotiden 6955 bis 8265 der Tabelle 5;
b) Nucleinsäuresequenzen, die mit den Nucleotiden aus a) unter hochstringenten Bedingungen hybridisieren; und
c) RNA-Sequenzen, transkribiert von den Nucleotiden aus a) oder b); und
ii) einer isolierten DNA-Sequenz, die mit einer DNA-Sequenz, die nicht typisierbares Haemopilus influenzae Serotyp 1 LKP Tip-Adhäsin-Protein kodiert, unter hochstringenten Bedingungen hybridisiert, wobei die DNA-Sequenz, die das Serotyp 1 LKP Tip-Adhäsin-Protein kodiert, aus den Nucleotiden 6955 bis 8265 von Tabelle 5 besteht.

2. Isoliertes, nicht typisierbares Haemophilus influenzae Protein, umfassend:
ein Serotyp 1 LKP Tip-Adhäsin-Protein mit der durch das ORF der Nucleotide 6955 bis 8265 von Tabelle 5 kodierten Aminosäuresequenz, und biologisch aktive Fragmente davon, die sich an native Erythrozyten-Ghost-Präparate binden, aber an denaturierte Erythrozyten-Ghosts nicht binden.

3. Rekombinanter Expressionsvektor, umfassend ein DNA-Insert, wobei das genannte DNA-Insert mit dem Komplement einer Nucleotidsequenz hybridisiert, die nicht typisierbares Haemophilus influenzae LKP Tip-Adhäsin-Protein unter hochstringenten Bedingungen kodiert, wobei die genannte Nucleotidsequenz aus den Nucleotiden 6955 bis 8265 der Tabelle 5 besteht, wobei der genannte Expressionsvektor ein nicht typisierbares Haemophilus influenzae Tip-Adhäsin-Protein in einer prokaryotischen oder eukaryotischen Zelle exprimiert.

4. Rekombinanter Expressionsvektor nach Anspruch 3, wobei das DNA-Insert ein Protein kodiert, umfassend die durch das ORF der Nucleotide 6955 bis 8265 der Tabelle 5 kodierten Aminosäuresequenz.

5. Prokaryotische oder eukaryotische Wirtszelle, transformiert mit einem Expressionsvektor nach Anspruch 3.

6. In-vitro-Verfahren zur Herstellung eines nicht typisierbaren *Haemphilus influenzae* Proteins nach Anspruch 2 unter Verwendung des rekombinanten Expressionsvektors aus Anspruch 3 in einer prokaryotischen oder eukaryotischen Wirtszelle, umfassend das Transfizieren der Wirtszelle mit dem rekombinanten Expressionsvektor und Halten der transfizierten Wirtszelle unter Bedingungen, die zur Expression von nicht typisierbaren *Haemophilus influenzae* LKP Pilusproteinen in der Wirtszelle geeignet sind.

## Revendications

1. Séquence d'acide nucléique isolée sélectionnée parmi le groupe consistant en :
(i) une séquence d'acide nucléique isolée sélectionnée parmi le groupe consistant en :
a) les nucléotides 6955 à 8265 du Tableau 5 ;
b) des séquences d'acide nucléique qui s'hybrident aux nucléotides de a) dans des conditions de haute stringence ; et
c) des séquences d'ARN transcrites à partir des nucléotides de a) ou des séquences de b) ; et
(ii) une séquence d'ADN isolée qui s'hybride à une séquence d'ADN codant pour une protéine à rôle d'adhésive de l'extrémité des pili LKP (pour *Long thicK Positive*) de sérotype 1 d'*Haemophilus influenzae* non typable dans des conditions de haute stringence, ladite séquence d'ADN codant pour la protéine à rôle d'adhésive des extrémités des pili LKP de sérotype 1 consistant en les nucléotides 6955 à 8265 du Tableau 5.

2. Protéine d'*Haemophilus influenzae* isolé non typable, qui comprend :
une protéine à rôle d'adhésive de l'extrémité des pili LKP de sérotype 1 dont la séquence d'acides aminés est codée par la phase de lecture ouverte (PLO) comprenant les nucléotides 6955 à 8265 du Tableau 5, et des fragments biologiquement actifs de celle-ci qui se lient à des préparations d'hématies pâles natives mais pas à des hématies pâles dénaturées.

3. Vecteur d'expression recombinant comprenant un insert d'ADN, dans lequel ledit insert d'ADN s'hybride au complément d'une séquence nucléotidique codant pour une protéine à rôle d'adhésive de l'extrémité des pili LKP de sérotype 1 d'*Haemophilus influenzae* non typable dans des conditions de haute stringence, ladite séquence nucléotidique consistant en les nucléotides 6955 à 8265 du Tableau 5 et ledit vecteur d'expression exprimant une protéine à rôle d'adhésive de l'extrémité des pili LKP de sérotype 1 d'*Haemophilus influenzae* non typable dans une cellule procaryote ou eucaryote.

4. Vecteur d'expression selon la revendication 3, dans lequel ledit insert d'ADN code pour une protéine qui comporte la séquence d'acides aminés codée par la PLO comprenant les nucléotides 6955 à 8265 du Tableau 5.

5. Cellule hôte procaryote ou eucaryote transformée par un vecteur d'expression selon la revendication 3.

6. Méthode *in vitro* pour la production d'une protéine d'*Haemophilus influenzae* non typable selon la revendication 2 au moyen du vecteur d'expression recombinant de la revendication 3 dans une cellule hôte procaryote ou eucaryote, qui comprend la transfection de la cellule hôte avec le vecteur d'expression recombinant et le maintien de la cellule hôte transfectée dans des conditions qui se prêtent à l'expression de protéines des pili LKP d'*Haemophilus influenzae* non typable dans la cellule hôte.
